# EUROPÄISCHE PATENTSCHRIFT

(11) **EP 2 560 745 B1**
(45) Veröffentlichungstag und Bekanntmachung des Hinweises auf die Patenterteilung: **01.04.2020**
(21) Anmeldenummer: 11717989.5
(22) Anmeldetag: 20.04.2011
(51) Int. Cl.: A61M 1/16, B01D 63/02, B01D 63/04

(54) **VERFAHREN UND VORRICHTUNG ZUR HERSTELLUNG EINES HOHLFASERMEMBRANMODULS**
PROCESS AND APPARATUS FOR PRODUCING A HOLLOW FIBRE MEMBRANE MODULE
PROCÉDÉ ET DISPOSITIF POUR PRODUIRE UN MODULE MEMBRANAIRE À FIBRES CREUSES

(30) Priorität: 20.04.2010 DE 102010027973
(43) Veröffentlichungstag der Anmeldung: 27.02.2013
(73) Patentinhaber: Xenios AG, 74076 Heilbronn (DE)
(72) Erfinder: STRAUSS, Andreas, 44801 Bochum (DE); GROSSER, Albert, 44801 Bochum (DE)
(74) Vertreter: Castell, Klaus
(86) Internationale Anmeldenummer: PCT/EP2011/056357
(87) Internationale Veröffentlichungsnummer: WO 2011/131734

(56) Entgegenhaltungen:
- WO-A1-83/00098
- US-A- 3 801 401
- US-A- 4 346 006
- US-A- 5 284 584

## Beschreibung

Die Erfindung betrifft ein Verfahren zur Herstellung eines Hohlfasermembranmoduls sowie eine Vorrichtung, die zur Durchführung dieses Verfahrens geeignet ist. Die Erfindung betrifft ferner ein Hohlfasermembranmodul.

Im Rahmen der Behandlung von Patienten mit akutem Lungenversagen (ARDS), schweren respiratorischen Erkrankungen oder pulmonalen Insuffizienzen, aber auch in der kardiopulmonalen Chirurgie übernimmt in vielen Fällen ein externes Gerät, beispielsweise eine künstliche Lunge, zeitweise die Funktionen der Lunge des Patienten. Ein wichtiger Bestandteil der künstlichen Lunge ist ein Oxygenator, in dem der erforderliche Gasaustausch zur Versorgung des Patienten mit Sauerstoff stattfindet.

Zu den verschiedenen Arten von Oxygenatoren gehören Filmoxygenatoren, Blasenoxygenatoren und Membranoxygenatoren, wobei in der modernen Medizin überwiegend nur noch Membranoxygenatoren im Verfahren der extrakorporalen Membranoxygenation (ECMO) zur Anwendung kommen.

Während einer ECMO-Behandlung wird dem Blut eines Patienten über einen Membranoxygenator lebenswichtiger Sauerstoff zugeführt und das durch Stoffwechselprozesse entstehende Kohlendioxid entfernt. Gas- und Blutseite sind dafür durch eine gasdurchlässige mikroporöse Membran voneinander getrennt und der Gasaustausch erfolgt durch Partialdruckdifferenzen entlang der Membran. Die Membranen sind häufig als semipermeable Hohlfasern ausgebildet, an denen vorzugsweise außen das Blut vorbeiströmt, während ein Sauerstoff-Luft-Gemisch die Hohlfasern von innen durchströmt. Gas- und Blutstrom können dabei beispielsweise im Gleich- oder Gegenstrom oder auch im Kreuzstrom zueinander geführt werden. An der Membran kommt es aufgrund eines Konzentrationsgefälles zum Austausch von Sauerstoff und Kohlendioxid. Das durchströmende Blut wird mit Sauerstoff angereichert und vom Kohlendioxid befreit. Die Trennung von Gas- und Blutseite ermöglicht eine Steuerung der Sauerstoffsättigung im Blut. Ein Membranoxygenator ist üblicherweise nur zum einmaligen Gebrauch bestimmt und wird nach der Anwendung entsorgt.

Das Kernstück eines Membranoxygenators oder auch Hohlfasermembranmoduls bildet ein Hohlfaserbündel mit einer oder mehreren Trennschichten, das aus einer Vielzahl von einzelnen Hohlfasern gebildet wird, die an den jeweiligen Enden über eine Vergussmasse miteinander verbunden sind und das von einem Gehäuse umschlossen wird. Allgemein sollte noch darauf verwiesen werden, dass derartige Hohlfaserbündel nicht nur in Membranoxygenatoren einer künstlichen Lunge Anwendung finden. Weitere mögliche Anwendungsgebiete für Hohlfaserbündel ergeben sich beispielsweise in der Dialyse oder auch in der Biotechnologie oder Pharmazie, beispielsweise zur Verarbeitung von Zellkulturen.

Für das Vergießen der Enden der Hohlfasern und das damit verbundene Ausbilden von vergossenen Randbereichen sind dabei insbesondere das Zentrifugalgießverfahren und das Tauchgießverfahren bekannt. Diese beiden Verfahren sind üblicherweise jedoch mit dem Nachteil verbunden, dass sie erst nach dem Aufwickeln der Matte aus Hohlfasern auf den Wickelkern durchgeführt werden können und dass dafür eine Übergabe an die Gießeinrichtungen erforderlich ist, bei der die Hohlfasern mechanisch geschädigt werden können. Zudem sind beide Verfahren sehr arbeitsintensiv und damit teuer.

In einem anderen, in der US 5 284 584 beschriebenen Verfahren zur Ausbildung vergossener Randbereiche und/oder Trennschichten werden deshalb ein oder mehrere Schmelzbänder einer Vergussmasse auf eine Matte mit Hohlfasern aufgebracht, während die Matte von einem Wickelkern abgewickelt und auf einen anderen Wickelkern aufgewickelt wird. In diesem Verfahren wird die Vergussmasse von einer Spritzmaschine aufbereitet und mit einer bandförmigen Düse als Schmelzband auf die Matte aufgebracht, bevor die Matte aufgewickelt wird, sodass eine Übergabe an eine nachfolgende Gießeinrichtung nicht mehr erforderlich ist.

Wickelkerne für die Herstellung von Membranmodulen aus Matten aus Hohlfasern mit zwei oder mehr Teilen sind aus US3801404 A, WO83/00098 A1, US4346006 A und US5284584 A bekannt.

Mit den bekannten Verfahren zum Vergießen der Enden der Hohlfasern und zum Einbringen der Trennschicht in das Hohlfaserbündel stehen jedoch nur begrenzte Möglichkeiten für die Ausgestaltung und Anordnung der Formen und Konturen für die vergossenen Randbereiche und für die Trennschicht des Hohlfaserbündels zur Verfügung. Insbesondere können mit diesen Verfahren nur gleichförmige, vergossene Bereiche ausgebildet werden, sodass die Variabilität für die Gestaltung der vergossenen Randbereiche und der Trennschicht entsprechend eingeschränkt ist.

Eine Aufgabe der Erfindung ist es daher, ein Verfahren bereitzustellen, mit dessen Hilfe Hohlfasermembranmodule hergestellt werden können, bei denen einerseits Formen und Konturen und andererseits Anzahl und Anordnung sowohl für die vergossenen Randbereiche als auch für die Trennschicht variabel gestaltet werden können.

Eine Aufgabe der Erfindung ist es ferner, eine Vorrichtung zur Durchführung eines solchen Verfahrens sowie ein Hohlfasermembranmodul selbst bereitzustellen.

Diese Aufgabe wird gelöst durch ein Verfahren mit den Merkmalen des unabhängigen Anspruchs 1. Vorteilhafte Weiterbildungen des Verfahrens ergeben sich aus den Unteransprüchen 2-8,

Die Aufgabe wird ferner durch eine Vorrichtung nach Anspruch 9 gelöst. Ausführungsformen der Vorrichtung ergeben sich aus den Unteransprüchen 10-17. Weiterhin wird die Aufgabe gelöst durch ein Hohlfasermembranmodul mit den Merkmalen des unabhängigen Anspruchs 18.

Dieses Verfahren hat einerseits den Vorteil, dass bereits mit dem Aufwickeln der mit Vergussmasse benetzten Matte ein Hohlfaserbündel mit vergossenen Bereichen ausgebildet wird. Zusätzliche Verfahrensschritte zum Vergießen der Enden der Hohlfasern nach dem Aufwickeln der Matte sind damit nicht mehr erforderlich. Andererseits hat dieses Verfahren den Vorteil, dass in Abhängigkeit von der ausgebildeten Benetzungskontur auf der Matte vergossene Bereiche beliebiger Form im Hohlfaserbündel ausgebildet werden können. Somit können vergossene Bereiche ausgebildet werden, deren Form an einen optimalen Fluss der zu verarbeitenden Flüssigkeiten, beispielsweise von Blut oder Zellkulturen, durch das Hohlfasermembranmodul mit geringstmöglichen Schädigungen der Bestandteile der Flüssigkeiten während des Durchflusses angepasst ist. Zudem gewährleisten die vergossenen Bereiche eine bessere Durchmischung der Flüssigkeiten während des Durchflusses sowie eine Erhöhung der lokalen Verweilzeiten der Flüssigkeiten, was wiederum zu einer Optimierung des Stoffaustauschs führt. Die Synchronisation der beiden Geschwindigkeiten stellt sicher, dass die Matte mit dem Ablageelement transportiert werden kann, ohne auf dem Ablageelement zu verrutschen oder sich zu verschieben.

Der Vorteil der Ausbildung des zweiten Wickelkerns aus mehreren, zusammengefügten Kernteilen liegt insbesondere darin, dass damit ein zweiter Wickelkern ohne Verbindungsstege ausgebildet werden kann, die üblicherweise bei einem einteiligen Wickelkern durch die dann erforderlichen Aussparungen für den Durchfluss der Flüssigkeiten ausgebildet werden. Dadurch können Flüssigkeiten, beispielsweise Blut oder Zellkulturen, partikel- oder teilchenschonend durch das fertige Hohlfasermembranmodul geleitet werden, da keine Verbindungsstege den Fluss der Flüssigkeiten behindern. Die Flüssigkeiten können somit frei und ungehindert durch das Hohlfasermembranmodul fließen und müssen nicht an mehreren Stegen vorbei geleitet werden. Positionierung und Ausrichtung der beiden Aufnahmen stellen sicher, dass die benetzte Matte gleichmäßig und ohne Verwerfungen oder Verschiebungen auf den zweiten Wickelkern aufgewickelt werden kann.

In einer Ausgestaltung umfasst das Verfahren folgende weitere Schritte:
- Verdichten der Vergussmasse auf der Matte und gleichzeitiges Entfernen überschüssiger Vergussmasse von der Matte;
   Das Verdichten und das Entfernen überschüssiger Vergussmasse gewährleisten eine gleichmäßige Verteilung der Vergussmasse sowie präzise Ausbildung der Benetzungskonturen auf der Matte.
- Abnehmen der benetzten Matte von dem Ablageelement;
   Dadurch wird verhindert, dass das Ablageelement in das Hohlfaserbündel mit eingewickelt wird.
- Aufwickeln der benetzten Matte (102) auf den zweiten Wickelkern (106), wobei die erste Aufnahme von einer ersten Antriebseinheit (124) angetrieben wird und wobei die zweite Aufnahme mit einer Drehzahl gedreht wird, die mit der von der ersten Antriebseinheit erzeugten Drehzahl der ersten Aufnahme synchronisiert ist;
   Mit dem Aufwickeln werden mehrere Lagen der benetzten Matte übereinander gelagert, wodurch erst die vergossenen Bereiche ausgebildet werden, deren Formen auf den Benetzungskonturen der Vergussmasse auf der Matte basieren. Die erste Antriebseinheit gewährleistet, dass die Matte mit der Geschwindigkeit vₘ bewegt wird und die Synchronisation gewährleistet, dass die Matte gleichmäßig auf den zweiten Wickelkern aufgewickelt wird.
- Überwachen bestimmter Maße des Hohlfaserbündels während des Aufwickelns der benetzten Matte;
- Stoppen des Aufwickelns, wenn das gewickelte Hohlfaserbündels definierte Abmessungen aufweist;
   Überwachung und Stopp des Aufwickelns stellen sicher, dass für das gewickelte Hohlfaserbündel definierte Abmessungen nach dem Beendigen des Wickelvorgangs eingehalten und nicht über- oder unterschritten werden.
- Trennen des gewickelten Hohlfaserbündels von der Matte.
   Durch dieses Trennen wird der Wickelvorgang abgeschlossen und das gewickelte Hohlfaserbündel kann weiter verarbeitet werden.

In einer anderen Ausgestaltung umfasst das Verfahren folgende weitere Schritte:
- Montieren eines hohlzylinderförmigen Gehäuses um das gewickelte Hohlfaserbündel, wobei das Gehäuse mindestens zwei zumindest bereichsweise voneinander getrennte Gehäuseteile umfasst, die das Hohlfaserbündel nach dem Montieren zumindest bereichsweise umschließen und wobei das Gehäuse an beiden Enden offen ist;
   Die Verwendung von zwei zumindest bereichsweise voneinander getrennten Gehäuseteilen ermöglicht eine Montage des Gehäuses ohne mechanische Schädigung der empfindlichen Hohlfasern beispielsweise durch Aufschieben eines zylinderförmigen Gehäuses, da die beiden Gehäuseteile nur um das gewickelte Hohlfaserbündel herum gelegt und nicht aufgeschoben werden müssen. Zudem wird durch die Verwendung von zwei Gehäuseteilen auch eine Randgängigkeit der durchgeleiteten Flüssigkeiten wirkungsvoll verhindert, da die beiden Gehäuseteile sehr eng um das Hohlfaserbündel gelegt werden können.
- Entnahme des Hohlfaserbündels mit dem Gehäuse, wobei die beiden Gehäuseteile fixiert werden;
- Vergießen der beiden Gehäuseteile mit Vergussmasse und Aushärten der Vergussmasse;
   Mit dem Vergießen der beiden Gehäuseteile wird sichergestellt, dass keine Flüssigkeiten unbeabsichtigt aus dem Gehäuse austreten können.
- Begradigen beider Randbereiche des gewickelten Hohlfaserbündels, sodass alle Hohlfasern jeweils offene Enden aufweisen.
   Damit wird erreicht, dass alle Hohlfasern des gewickelten Hohlfaserbündels gleichmäßig angeströmt werden können und dass beispielsweise keine Verwirbelungen in den Gasströmen im Ein- und Austrittsbereich der Hohlfasern entstehen.

In einer Ausgestaltung umfasst das Verfahren folgende weitere Schritte vor dem Schritt des Transportes der Matte:
- Befestigen eines Endes von mindestens einem bandförmigen Führungselement an dem zweiten Wickelkern;
- Befestigen des anderen Endes des mindestens einen Führungselementes an einem Anfangsbereich der Matte;
   Das Führungselement unterstützt insbesondere den positionsgenauen Transport des Mattenanfangs vom ersten zum zweiten Wickelkern. Zudem kann mit der Verwendung des Führungselementes das Benetzen bereits am Mattenanfang begonnen werden und das anschließende Aufwickeln des benetzten Mattenanfangs auf den zweiten Wickelkern wird mit dem Führungselement entsprechend vereinfacht.
- Auflegen der Matte auf das bandförmige Ablageelement;
- Vorspannen der Matte und zumindest teilweises Aufwickeln des Führungselementes auf den zweiten Wickelkern;
   Mit dem Vorspannen der Matte wird einerseits eine genaue Ausrichtung der Matte auf dem Ablageelement erreicht und andererseits eine genaue Ausbildung der Benetzungskonturen auf der Matte unterstützt.
- Abwickeln der Matte von dem ersten Wickelkern.

Eine Ausgestaltung des Verfahrens sieht vor, dass das Aufwickeln der benetzten Matte in einer Kammer unter einer von einem Niederdruck-Plasmagenerator erzeugten Atmosphäre erfolgt, sodass das Hohlfaserbündel während des Aufwickelns gleichzeitig sterilisiert wird. In einer Weiterbildung des Verfahrens wird das gewickelte und sterilisierte Hohlfaserbündel automatisch in der Kammer in das Gehäuse montiert und steril verpackt. Damit entfallen sowohl separate Sterilisationsschritte als auch vorgeschriebene Ausgasungszeiten bei Sterilisation mit einem Gas wie beispielsweise Ethylenoxid (EO), sodass das Hohlfasermembranmodul nach der Herstellung sofort und ohne Einschränkungen verwendbar ist.

In einer Ausgestaltung des Verfahrens wird das Auflageelement von einem dritten, zylinderförmigen Wickelkern abgewickelt und auf einen vierten, zylinderförmigen Wickelkern aufgewickelt, wobei der vierte Wickelkern von einer zweiten Antriebseinheit angetrieben wird. In einer anderen Ausgestaltung wird das Auflageelement von dem dritten, zylinderförmigen Wickelkern über eine Andruckrolle weitergeführt, wobei diese Andruckrolle von der zweiten Antriebseinheit angetrieben wird. Das Ablageelement ist hierbei nur einmal verwendbar und kann nach der Verwendung entsprechend entsorgt werden, sodass damit die hohen Anforderungen an die Reinheit der Hohlfasermembranmodule erfüllt werden können. Die zweite Antriebseinheit für den vierten Wickelkern stellt sicher, dass das Ablageelement mit der Fördergeschwindigkeit v_{f} bewegt wird.

In einer anderen Ausgestaltung des Verfahrens wird das Auflageelement als Endlosband von mindestens einer Antriebswelle bewegt und um mindestens eine weitere Welle geführt, wobei das Auflageelement nach der Abnahme der benetzten Matte gereinigt wird. Damit wird sichergestellt, dass Verunreinigungen der nicht benetzten Matte bei erneuter Verwendung des Ablageelementes vermieden werden. Hier stellt die Antriebswelle sicher, dass das Ablageelement mit der Fördergeschwindigkeit v_{f} bewegt wird.

In einer Ausgestaltung umfasst die Vorrichtung weiterhin:
- eine Überwachungseinrichtung, die so ausgebildet ist, dass sie bestimmte Maße des Hohlfaserbündels während des Aufwickelns der benetzten Matte überwachen kann und dass sie das Aufwickeln stoppen kann, wenn das gewickelte Hohlfaserbündel definierte Abmessungen aufweist; sowie
- eine Trenneinrichtung, die so ausgebildet ist, dass sie das gewickelte Hohlfaserbündel von der Matte trennen kann.

In einer weiteren Ausgestaltung umfasst die Vorrichtung weiterhin:
- eine Montageeinrichtung, die so ausgebildet ist, dass sie ein hohlzylinderförmiges Gehäuse um das gewickelte Hohlfaserbündel montieren kann, wobei das Gehäuse mindestens zwei zumindest bereichsweise voneinander getrennte Gehäuseteile umfasst, die das Hohlfaserbündel nach der Montage zumindest bereichsweise umschließen und wobei das Gehäuse an beiden Enden offen ist;
- eine Entnahmeeinrichtung, die so ausgebildet ist, dass sie das Hohlfaserbündel mit dem Gehäuse entnehmen kann;
- eine Vergusseinrichtung für Vergussmasse, die so ausgebildet ist, dass sie die beiden Gehäuseteile des Gehäuses vergießen kann;
- eine Aushärteeinrichtung, die so ausgebildet ist, dass sie die Vergussmasse aushärten kann; sowie
- eine Schneideinrichtung, die so ausgebildet ist, dass sie beide Randbereiche des gewickelten Hohlfaserbündels so begradigen kann, dass alle Hohlfasern jeweils offene Enden aufweisen.

In einer weiteren Ausgestaltung umfasst die Vorrichtung weiterhin:
- eine Zuführeinrichtung, die so ausgebildet ist, dass sie die Matte von einem ersten Wickelkern abwickeln kann; sowie
- eine Spanneinrichtung, die so ausgebildet ist, dass sie die Matte vorspannen kann.

In einer Weiterbildung der Vorrichtung weist die Dosiereinrichtung ein Rakelelement auf, das so ausgebildet ist, dass es die Vergussmasse auf der Matte verdichten und gleichzeitig überschüssige Vergussmasse von der Matte entfernen kann. Dieses Rakelelement gewährleistet eine gleichmäßige Verteilung der Vergussmasse sowie eine präzise Ausbildung der Benetzungskonturen auf der Matte.

In einer anderen Weiterbildung der Vorrichtung weist die Überwachungseinrichtung einen Sensor zur Messung eines Durchmessers des gewickelten Hohlfaserbündels auf. Mit dem Sensor kann der Durchmesser des Hohlfaserbündels während des Aufwickelns der benetzten Matte permanent überwacht werden, sodass sichergestellt ist, dass der Wickelvorgang beendet wird, wenn das Hohlfaserbündel einen vorgegebenen Durchmesser erreicht hat.

In einer Ausgestaltung der Vorrichtung ist die Entnahmeeinrichtung zangenförmig ausgebildet und weist mindestens ein Fixierelement zum Fixieren der beiden Gehäuseteile während der Entnahme auf. Die Zangenform ermöglicht eine passgenaue Aufnahme des gewickelten Hohlfaserbündels mit dem Gehäuse und das Fixierelement stellt sicher, dass die beiden Gehäuseteile in ihrer Position verbleiben, wenn Hohlfaserbündel mit Gehäuse entnommen werden.

Bei einer Ausgestaltung der Vorrichtung ist die Wickeleinrichtung in einer Kammer angeordnet, wobei die Kammer so ausgebildet ist, dass in der Kammer mittels eines Niederdruck-Plasmagenerators eine Atmosphäre zum Sterilisieren des Hohlfaserbündels während des Aufwickelns erzeugbar ist. In einer Weiterbildung der Vorrichtung ist in der Kammer eine automatische Gehäusemontage- und Verpackungseinrichtung für das gewickelte und sterilisierte Hohlfaserbündel angeordnet. Damit entfällt der separate Vorgang der Sterilisation des Hohlfasermembranmoduls.

Die Erfindung sieht ferner ein Hohlfasermembranmodul vor, das nach dem Verfahren hergestellt wurde.

Das erfindungsgemäße Hohlfasermembranmodul zeichnet sich insbesondere dadurch aus, dass die darin ausgebildeten vergossenen Bereiche eine beliebige gleichförmige oder ungleichförmige Form aufweisen können und dass Anzahl und Anordnung der vergossenen Bereiche variabel gestaltet werden können. Zudem entfällt der separate Verfahrensschritt des Vergießens der Randbereiche, da die vergossenen Bereiche bereits mit dem Aufwickeln der Matte ausgebildet werden. Dadurch wird auch das Risiko minimiert, dass die Hohlfasern des gewickelten Hohlfaserbündels während der Übergabe an die nachfolgende Vergusseinrichtung mechanisch beschädigt werden. Anwendungsgebiete für das erfindungsgemäße Hohlfaserbündel finden sich beispielsweise in der Herzchirurgie als Oxygenator, in der Dialyse als Dialysator oder auch in der Biotechnologie oder Pharmazie, beispielsweise zur Verarbeitung von Zellkulturen.

Die zuvor genannten und weitere Vorteile, Besonderheiten und zweckmäßige Weiterbildungen der Erfindung werden auch anhand der Ausführungsbeispiele deutlich, die nachfolgend unter Bezugnahme auf die Figuren beschrieben werden.

Von den Figuren zeigt:

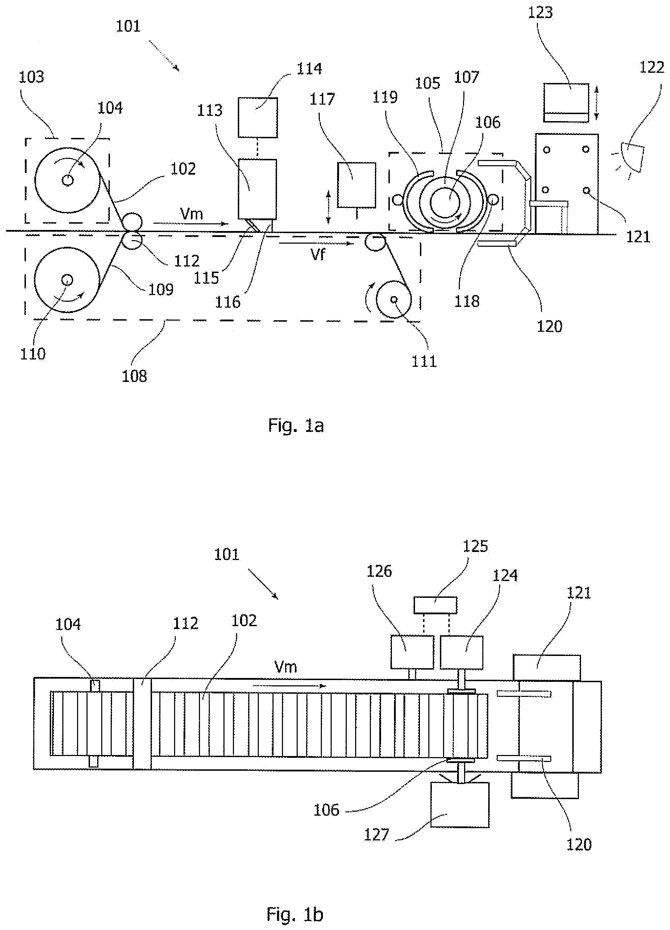

- Fig. 1 a: eine Seitenansicht einer Vorrichtung zur Herstellung eines Hohlfasermembranmoduls;
- Fig. 1: b eine Draufsicht dieser Vorrichtung;
- Fig. 2a: eine maschinelle Aufnahme einer Wickeleinrichtung für einen mehrteiligen Wickelkern;
- Fig. 2b: eine manuelle Aufnahme einer Wickeleinrichtung für einen mehrteiligen Wickelkern;
- Fig. 3a: eine Hohlfasermatte;
- Fig. 3b: eine bereichsweise benetzte Hohlfasermatte;
- Fig. 4a: ein gewickeltes Hohlfaserbündel mit einem zweiteiligen Gehäuse;
- Fig. 4b: ein halbschalenförmiges Gehäuseteil;
- Fig. 5: eine Seitenansicht einer anderen Vorrichtung zur Herstellung eines Hohlfasermembranmoduls; sowie
- Fig. 6: eine Seitenansicht der Vorrichtung mit einer Kammer.

In Fig. 1a ist schematisch als Beispiel die Seitenansicht einer Vorrichtung 101 zur Herstellung eines Hohlfasermembranmoduls aus einer bandförmigen Matte 102 dargestellt. Die Vorrichtung 101 weist eine Zuführeinrichtung 103 für die Matte 102 auf, die auf einem ersten Wickelkern 104 aufgewickelte ist. Der Wickelkern 104 wird in der Zuführeinrichtung 103 gelagert und diese Lagerung sollte so ausgeführt sein, dass ein einfaches und leichtes Abwickeln der Matte 102 von dem ersten Wickelkern 104 möglich ist. Derartige Lagerungsmöglichkeiten sind dem Fachmann hinlänglich bekannt und werden hier nicht weiter beschrieben. Entsprechendes gilt auch für die Lagerung aller weiteren Wickelkerne. Es ist zu beachten, dass nicht nur eine einlagige Matte 102, sondern auch eine mehrlagige Matte 102 aufgewickelt werden kann. Ebenfalls möglich erscheint die Verwendung einzelner Hohlfasern, die erst zu einer Matte 102 verarbeitet werden, bevor die weitere Verarbeitung erfolgt.

Ein weiterer Bestandteil der Vorrichtung 101 ist eine Wickeleinrichtung 105, in der ein zweiter Wickelkern 106 auf einer Aufnahme der Wickeleinrichtung 105 gelagert ist, auf den die Matte 102 aufgewickelt und so ein gewickeltes Hohlfaserbündel 107 ausgebildet werden kann. Zum Aufwickeln wird die Matte 102 mit einer Geschwindigkeit vₘ vom ersten Wickelkern 104 zum zweiten Wickelkern 106 bewegt. Die Bewegungsrichtung der Matte 102 ist mit einem Pfeil entsprechend gekennzeichnet.

Der zweite Wickeikern 106 kann sowohl einteilig als auch mehrteilig ausgeführt sein. Bei einem einteiligen, zweiten Wickelkern 106, der mit mehreren Aussparungen versehen ist, sodass sich einzelne Verbindungsstege ergeben, wird dieser einfach auf der Aufnahme der Wickeleinrichtung 105 gelagert und anschließend kann mit dem Wickeln begonnen werden. Ein mehrteiliger, zweiter Wickelkern 106 hingegen erfordert ein vorheriges Zusammenfügen der einzelnen Teile, was weiter unten detailliert beschrieben wird.

Die Vorrichtung 101 weist weiterhin eine Fördereinrichtung 108 für ein bandförmiges Ablageelement 109 auf, das auf einen dritten Wickelkern 110 aufgewickelt ist. Der Wickelkern 110 wird in der Fördereinrichtung 108 gelagert und auch diese Lagerung sollte so ausgeführt sein, dass ein einfaches und leichtes Abwickeln des bandförmigen Ablageelementes 109 von dem dritten Wickelkern 110 möglich ist. In der Fördereinrichtung 108 ist noch ein vierter Wickelkern 111 gelagert, auf den das Ablageelement 109 aufgewickelt werden kann. Zum Aufwickeln wird das Ablageelement 109 mit einer Fördergeschwindigkeit v_{f} vom dritten Wickelkern 110 zum vierten Wickelkern 111 bewegt. Die Bewegungsrichtung des Ablageelementes 109 ist ebenfalls mit einem Pfeil entsprechend gekennzeichnet. Alternativ kann das Ablageelement 109 auch von dem dritten Wickelkern 110 abgewickelt und über eine Andruckrolle beispielsweise in einen Ablagebehälter weitergeführt werden.

Bei dem Ablageelement 109 handelt es sich beispielsweise um eine dünne Folie, auf die die Matte 102 aufgelegt werden kann und die so flexibel ist, dass sie auf den dritten Wickelkern 110 aufgewickelt werden kann. Als Material können verschiedene Kunststoffe, wie beispielweise Polyethylen (PE), Polypropylen (PP), Polyvinylchlorid (PVC), Polystyrol (PS) oder Polycarbonat (PC) verwendet werden. Es können aber auch andere Materialien gewählt werden. Hauptkriterium bei der Auswahl des Materials ist zum einen, dass die Matte 102 durch das Ablageelement 109 nicht verunreinigt oder kontaminiert wird. Zum anderen sind es niedrige Kosten, da das Ablageelement 109 in dieser Ausführungsform der Vorrichtung 101 nur für den einmaligen Gebrauch ausgelegt ist und nach dem Gebrauch entsorgt wird.

Zum Vorspannen von Matte 102 und Ablageelement 109 ist in die Vorrichtung 101 eine Spanneinrichtung 112 integriert. Diese Spanneinrichtung 112 kann beispielsweise aus einer oder mehreren federnd gelagerten Wellen gebildet werden, um die entweder die Matte 102 oder das Ablageelement 106 entsprechend geführt werden. Ebenfalls möglich ist die Verwendung von federnd gelagerten Andruckelementen, wie beispielsweise Rollen.

Die Vorrichtung 101 weist eine Dosiereinrichtung 113 zum Dosieren von Vergussmasse auf der Matte 102 auf. Diese Dosiereinrichtung 113 ist mit einer Steuerungseinrichtung 114 verbunden, die vorgibt, mit weichen Mengen Vergussmasse die Matte 102 an welchen Positionen benetzt werden soll. Für das Benetzen der Matte 102 mit Vergussmasse umfasst die Dosiereinrichtung 113 eine Düse 115, die entsprechend den Vorgaben der Steuerungseinrichtung 114 oberhalb der Matte 102 positioniert werden kann. Die Dosiereinrichtung 113 umfasst außerdem ein Rakelelement 116, das zum einen die mit der Düse 115 auf die Matte 102 aufgebrachte Vergussmasse auf der Matte 102 verdichtet und das zum anderen überschüssige Vergussmasse von der Matte 102 abstreift. Alternativ zur Positionierung der Düse 115 oberhalb der Matte 102 kann auch die Matte 102 selbst unter einer feststehenden Düse so bewegt werden, dass die Matte 102 an den vorgegebenen Positionen mit Vergussmasse benetzt wird.

Zum Trennen des Hohlfaserbündels 107 von der Matte 102 ist in der Vorrichtung 101 eine Trenneinrichtung 117 vorgesehen. Diese Trenneinrichtung 117 kann beispielsweise als Schneidwerkzeug ausgebildet sein, mit dem die Matte 102 durchschnitten wird, wenn das Aufwickeln der Matte 102 auf den zweiten Wickelkern 106 abgeschlossen ist. Es sind aber auch andere Ausgestaltungen der Trenneinrichtung 117 vorstellbar. Die Trenneinrichtung 117 ist in der Vorrichtung 101 verfahrbar, sodass sie nur dann in Richtung der Matte 102 bewegt wird, wenn die Matte 102 vom Hohlfaserbündel 107 getrennt werden soll. Nach dem Trennen wird die Trenneinrichtung 117 wieder von der Matte 102 weg bewegt, um Störungen während des Aufwickelns der Matte 102 für ein nächstes Hohlfaserbündel 107 zu vermeiden. Der Verfahrweg der Trenneinrichtung 117 ist in dieser Ausführungsform mit einem vertikalen Pfeil gekennzeichnet. Ein vertikaler Verfahrweg ist jedoch nicht zwingend, der Verfahrweg kann auch horizontal oder diagonal verlaufen.

In die Vorrichtung 101 ebenfalls integriert ist eine Montageeinrichtung 118, in der zwei halbschalenförmige Gehäuseteile 119 um das gewickelte Hohlfaserbündel 107 montiert werden können. Diese Montageeinrichtung 118 kann beispielsweise als automatische Zuführeinheit ausgebildet sein, mit der die beiden Gehäuseteile 119 zugeführt und um das gewickelte Hohlfaserbündel 107 herum so angeordnet werden, dass ein geschlossenes Gehäuse um das Hohlfaserbündel 107 ausgebildet wird. Die Montageeinrichtung 118 muss jedoch nicht zwingend automatisch ausgestaltet sein. Die beiden Gehäuseteile 119 können auch halbautomatisch oder manuell montiert werden. Weiterhin ist eine Ausgestaltung des Gehäuses als aufklappbares Gehäuse möglich. Vorstellbar ist zudem, dass mehr als zwei einzelne Gehäuseteile verwendet werden.

Für die Entnahme des gewickelten Hohlfaserbündels 107 mit den beiden montierten Gehäuseteilen 119 ist in der Vorrichtung 101 eine Entnahmeeinrichtung 120 vorgesehen. Die Entnahmeeinrichtung 120 wird in dieser Ausführungsform beispielsweise aus zwei automatisch angesteuerten Grippzangen gebildet, die das Gehäuse mit dem Hohlfaserbündel 107 jeweils im Seitenbereich positionsgenau umfassen und entnehmen können. Die beiden Grippzangen sind dabei so ausgestaltet, dass sie die beiden Gehäuseteile 119 während der Entnahme fixieren, sodass sie sich nicht auf dem Hohlfaserbündel 107 verschieben können. Dafür weisen die Grippzangen Fixierelemente, wie beispielsweise Feststellschrauben oder auch Scheiben auf, die eine Veränderung der Position der beiden Gehäuseteile 119 während der Entnahme verhindern. Die Entnahmeeinrichtung 120 muss jedoch nicht zwingend automatisch ausgestaltet sein. Das Gehäuse mit dem Hohlfaserbündel 107 kann beispielsweise auch mit entsprechend ausgebildeten Zangen als Entnahmeeinrichtung 120 halbautomatisch oder manuell entnommen werden. Auch eine Ausbildung der Entnahmeeinrichtung 120 als Grippzangen ist nicht zwingend. Es können auch andere Entnahmeeinrichtungen 120 für eine positionsgenaue Entnahme des Gehäuses mit dem Hohlfaserbündel 107 verwendet werden, die dem Fachmann bekannt sind und hierfür geeignet erscheinen.

Zum Verbinden der beiden Gehäuseteile 119 weist die Vorrichtung 101 eine Vergusseinrichtung 121 für eine Vergussmasse auf, die den beiden Gehäuseteilen 119 über nicht dargestellte Düsen zugeführt wird. Zum Aushärten dieser Vergussmasse ist in der Vorrichtung eine Aushärteeinrichtung 122 vorgesehen, die in Abhängigkeit von der verwendeten Vergussmasse beispielsweise als Ofen oder Wärmelampe für warmaushärtende Vergussmassen oder als UV-Lampe für UV-aushärtende Vergussmassen ausgebildet sein kann.

Damit die einzelnen Hohlfasern der Matte 102 jeweils an beiden Enden offen sind und damit es im Ein- und Ausgangsbereich der Hohlfasern des Hohlfaserbündels 107 nicht zu Verwirbelungen in den Gasströmen kommt, umfasst die Vorrichtung 101 noch eine Schneideinrichtung 123 zum Begradigen der beiden vergossenen Randbereiche des gewickelten Hohlfaserbündels 107, die beispielsweise als Messer ausgestaltet ist. Dabei kann das Begradigen sowohl gerade als auch schräg erfolgen, um eine größtmögliche Öffnung der einzelnen Hohlfasern zu erreichen. Die Schneideinrichtung 123 ist in der Vorrichtung 101 verfahrbar, sodass sie nur dann in Richtung des Hohlfaserbündels 107 bewegt wird, wenn das Gehäuse um das Hohlfaserbündel 107 montiert und die Vergussmasse ausgehärtet ist. Nach dem Begradigen wird die Schneideinrichtung 123 wieder weg bewegt, um Störungen während der Montage eines nächsten Gehäuses zu vermeiden. Der Verfahrweg der Schneideinrichtung 123 ist in dieser Ausführungsform mit einem vertikalen Pfeil gekennzeichnet. Ein vertikaler Verfahrweg ist jedoch nicht zwingend, der Verfahrweg kann auch horizontal oder diagonal verlaufen.

Fig. 1b zeigt beispielhaft eine Draufsicht auf die Vorrichtung 101. An der Seite der Vorrichtung 101 ist eine erste Antriebseinheit 124 für die Aufnahme mit dem zweiten Wickelkern 106 angeordnet, die den zweiten Wickelkern 106 in eine Drehbewegung versetzen kann. Bedingt durch die Drehbewegungen des zweiten Wickelkerns 106 wird die Matte 102 vom ersten Wickelkern 104 abgewickelt und auf den zweiten Wickelkern 106 aufgewickelt. Die Antriebseinheit 124 ist mit einer Regelungseinrichtung 125 verbunden, die die erforderliche Drehzahl nₘ für die Antriebseinheit 124 vorgibt. Diese Drehzahl nₘ resultiert aus der Geschwindigkeit vₘ, mit der die Matte 102 vom ersten Wickelkern 104 zum zweiten Wickelkern 106 bewegt werden soll.

Ebenfalls an der Seite der Vorrichtung 101 ist eine zweite Antriebseinheit 126 für den vierten Wickelkern 111 angeordnet, die den vierten Wickelkern 111 in eine Drehbewegung versetzen kann. Bedingt durch die Drehbewegung des vierten Wickelkerns 111 wird das Ablageelement 109 vom dritten Wickelkern 110 abgewickelt und auf den vierten Wickelkern 111 aufgewickelt. Auch die Antriebseinheit 126 ist mit der Regelungseinrichtung 125 verbunden, die die erforderliche Drehzahl n_{f} für die Antriebseinheit 126 vorgibt. Diese Drehzahl n_{f} resultiert aus der Fördergeschwindigkeit v_{f}, mit der das Ablageelement 109 vom dritten Wickelkern 110 zum vierten Wickelkern 111 bewegt werden soll. Alternativ kann die Antriebseinheit 126 auch eine Andruckrolle antreiben, mit der das Ablageelement 109 nach dem Abwickeln vom dritten Wickelkern 110 weitergeführt und beispielsweise in einem Ablagebehälter abgelegt wird.

Auf der anderen Seite der Vorrichtung 101 ist eine Überwachungseinrichtung 127 angeordnet, mit der der Durchmesser des gewickelten Hohlfaserbündels 107 überwacht wird. Dafür weist die Überwachungseinrichtung 127 beispielsweise einen Sensor auf, der kontinuierlich den Durchmesser des Hohlfaserbündels 107 während des Aufwickelns der Matte 102 misst und ein Signal an die Überwachungseinheit 127 ausgibt, wenn das gewickelte Hohlfaserbündel 107 einen vorab definierten Durchmesser erreicht hat. Die Überwachungseinheit 127 kann aber beispielsweise auch einen mechanischen Anschlag aufweisen, mit dem ein Signal an die Überwachungseinheit 127 ausgelöst wird, wenn das Hohlfaserbündel 107 einen Durchmesser erreicht hat, bei dem die äußeren Hohlfasern den Anschlag berühren. Andere, dem Fachmann bekannte Ausgestaltungen sind ebenfalls möglich.

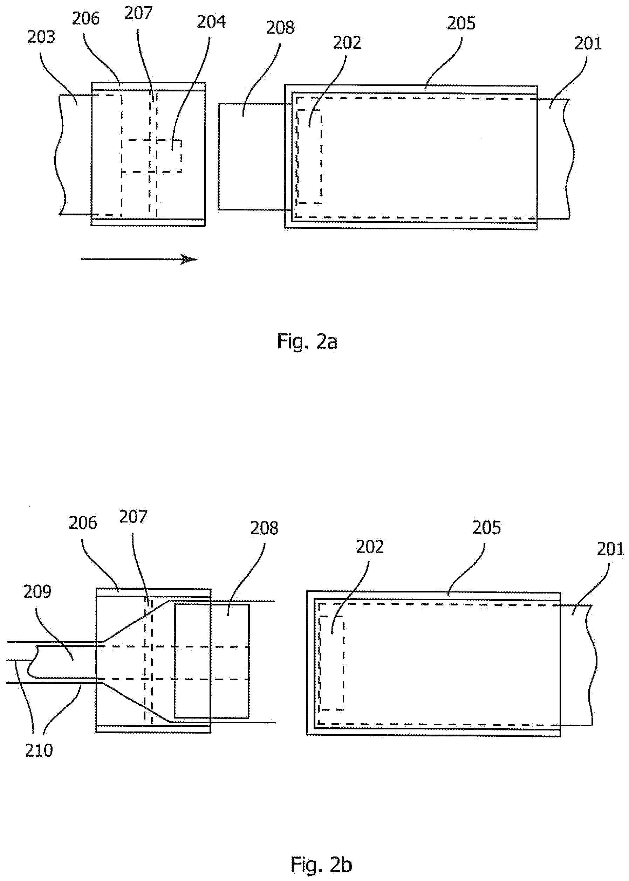

In Fig. 2a ist beispielhaft eine maschinelle Aufnahme der Wickeleinrichtung 105 für das Zusammenfügen eines mehrteiligen, zweiten Wickelkerns 106 dargestellt. Die Aufnahme umfasst eine erste Aufnahme 201 mit einem Magneten 202 und eine zweite Aufnahme 203 mit einem Zentrierelement 204 mit einer Zentrierspitze, wobei auf der ersten Aufnahme 201 das erste Kernteil 205 und auf der zweiten Aufnahme 203 das zweite Kernteil 206 montiert ist. Dabei wird das zweite Kernteil 206 über das Zentrierelement 204 auf der zweiten Aufnahme 203 zentriert. Das zweite Kernteil 206 ist als zylinderförmige Röhre ausgebildet, die im Inneren eine Einlasskontur 207 mit einer Bohrung aufweist, die zur Zentrierung des zweiten Kernteiles 206 auf dem Zentrierelement 204 verwendet wird. Das erste Kernteil 205 ist ebenfalls als zylinderförmige Röhre ausgebildet, die an einem Ende verschlossen ist. Auf der ersten Aufnahme 201 ist das erste Kernteil 205 so montiert, dass das geschlossene Ende über dem Magneten 202 angeordnet ist. Alternativ können die Kernteile 205 und 206 beispielsweise auch konisch oder mit anderen geometrischen Formen ausgebildet sein. Dann sind die beiden Aufnahmen 201 und 203 entsprechend an diese Formen angepasst.

Nach der Montage des ersten Kernteiles 205 und des zweiten Kernteiles 206 auf die jeweils zugehörige Aufnahme 201, 203 wird noch ein drittes, magnetisches Kernteil 208 auf dem Magneten 202 der ersten Aufnahme 201 positioniert. Bei diesem dritten Kernteil 208 handelt es sich beispielsweise um einen Rotor für eine Blutpumpe, mit dem Blut oder auch andere Flüssigkeiten durch das Hohlfasermembranmodul geleitet werden können. Der dreiteilige Aufbau des zweiten Wickelkerns 106 hat insbesondere den Vorteil, dass zwischen dem ersten Kernteil 205 und dem zweiten Kernteil 206 keine Verbindungsstege erforderlich sind, die beim Durchströmen des Hohlfasermembranmoduls mit Blut beispielsweise zur Schädigung der Blutbestandteile führen können.

Wenn die einzelnen Kernteile des zweiten Wickelkerns 106 auf der Aufnahme positioniert und ausgerichtet sind, wird die zweite Aufnahme 203 in Richtung der ersten Aufnahme 201 verfahren, bis die Zentrierspitze das dritte Kernteil 208 berührt, und wird dann axial ausgerichtet. Die Verfahrrichtung ist mit einem Pfeil entsprechend gekennzeichnet. Mit dieser Ausrichtung wird zum einen erreicht, dass das dritte Kernteil 208 positionsgenau zwischen dem ersten Kernteil 205 und dem zweiten Kernteil 206 angeordnet ist. Zum anderen wird damit gewährleistet, dass die Matte 102 ohne Verwerfungen auf den zweiten Wickelkern 106 aufgewickelt werden kann.

Nach dem Zusammenfügen des zweiten, mehrteiligen Wickelkerns 106 wird die Matte 102 auf den zweiten Wickelkern aufgewickelt. Dafür wird die erste Aufnahme 201 von der ersten Antriebseinheit 124 in eine Drehbewegung versetzt. Diese Drehbewegung wird über ein Synchronisationsgetriebe, beispielsweise über ein Zahnradgetriebe oder über eine Königswelle, an die zweite Aufnahme 203 übertragen, sodass Verwerfungen der Matte 102 auf dem zweiten Wickelkern 106 vermieden werden. Nach dem Wickeln sind die einzelnen Kernteile des zweiten Wickelkerns 106 dann fest positioniert und sowohl axial als auch radial zueinander fixiert. Eine weitere Erhöhung der Stabilität des Wickelkerns 106 kann noch durch die Verwendung eines Klebers erreicht werden.

Alternativ zum maschinellen Zusammenführen der einzelnen Kernteile des zweiten Wickelkerns 106 können diese Teile auch manuell zusammengefügt werden. In Fig. 2b ist schematisch eine manuelle Aufnahme zum Zusammenfügen des mehrteiligen, zweiten Wickelkerns 106 dargestellt. Die Aufnahme umfasst ebenfalls eine erste Aufnahme 201 mit einem Magneten 202, auf die das erste Kernteil 205 montiert ist.

Für das Zusammenfügen der Kernteile des zweiten Wickelkerns 106 wird durch die Bohrung der Einlasskontur 207 des zweiten Kernteiles 206 ein Montagestift 209 geführt, der an drei Seiten abgeflacht ist. Diese nicht dargestellten Abflachungen sind jeweils in einem Winkel von näherungsweise 120° um den Montagestift 209 herum angeordnet. An diesen Abflachungen entlang wird eine Montagehilfe 210 so in Richtung des dritten Kernteiles 208 geführt, dass sich das dritte Kernteil 208 nach der Montage in einer definierten Position im Inneren des zweiten Kernteiles 206 befindet. Die Montagehilfe 210 weist drei Beine auf und ist aus einem flexiblen Material, beispielsweise aus einem flexiblen Kunststoff, ausgebildet. Gleichfalls an den Abflachungen des Montagestiftes 209 entlang werden in Richtung des dritten Kernteiles 208 ebenfalls nicht dargestellte Montagefäden geführt, die anschließend um Teile des dritten Kernteiles 208 geführt werden und mit denen das dritte Kernteil 208 in das Innere des zweiten Kernteiles 206 gezogen wird. Bei den Montagefäden handelt es sich beispielsweise um Nylonfäden. Alternativ können beispielsweise aber auch dünne und flexible Drähte als Montagefäden verwendet werden.

Sowohl die Anzahl der Abflachungen des Montagestiftes 209 als auch damit verbunden die Anzahl der Beine der Montagehilfe 210 sowie der Montagefäden ist jedoch nicht auf drei beschränkt. Es können auch mehr oder weniger Abflachungen am Montagestift 209 angeordnet sein. Nach dem Positionieren des dritten Montageteiles 208 im Inneren des zweiten Kernteiles 206 wird das dritte Kernteil 208 auf dem ersten Kernteil 205 positioniert und dort mittels des Magneten 202 gehalten. Anschließend kann die Matte 102 auf den zusammengefügten, zweiten Wickelkern 106 aufgewickelt werden. Wenn das Wickeln maschinell erfolgen soll, wird vor dem Wickeln die erste Aufnahme 201 entsprechend entfernt und der zweite Wickelkern 106 in eine Aufnahme der Wickeleinrichtung 105 montiert. Montagestift 209, Montagehilfe 210 und Montagefäden werden nach dem Aufwickeln der Matte 102 wieder entfernt.

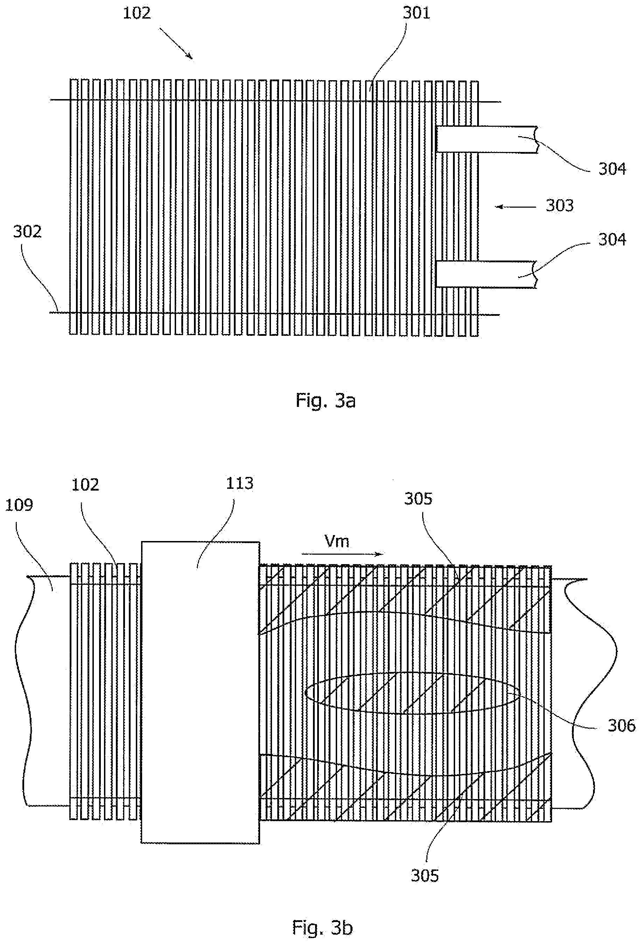

Für die Herstellung eines Hohlfasermembranmoduls wird eine bandförmige Matte 102 aus einzelnen, nebeneinander angeordneten Hohlfasern 301 verwendet, die mit Verbindungsfäden 302 miteinander verbunden sind. In Fig. 3a ist das Beispiel einer bandförmigen Matte 102 dargestellt. Verfahren zur Herstellung derartiger Matten sind im Stand der Technik hinreichend bekannt und werden hier nicht näher beschrieben.

Zur Vorbereitung des Verfahrens zur Herstellung eines Hohlfasermembranmoduls wird das Ablageelement 110 in die Fördereinrichtung 108 eingebracht. Dafür ist das Ablageelement 109 auf einem dritten Wickelkern 110 aufgewickelt, der in die dafür vorgesehene Lagerung der Fördereinrichtung 108 eingesetzt und dort arretiert wird. Anschließend wird das Ablageelement 109 teilweise vom dritten Wickelkern 110 abgewickelt und über die Spanneinrichtung 112 zum vierten Wickelkern 111 geführt und dort befestigt. Damit wird eine Verbindung zwischen dem dritten Wickelkern 110 und dem vierten Wickelkern 11 geschaffen, sodass das Ablageelement 109 vom dritten Wickelkern 110 abgewickelt und auf den vierten Wickelkern 111 aufgewickelt werden kann, wenn der vierte Wickelkern 111 von der Antriebseinheit 126 gedreht wird. Alternativ kann das Ablageelement 109 auch von einer Andruckrolle weitergeführt und nachfolgend in einem Ablagebehälter abgelegt werden.

Die Matte 102 ist auf einem ersten Wickelkern 104 aufgewickelt, der in die dafür vorgesehene Lagerung der Zuführeinrichtung 103 eingesetzt und dort arretiert wird. In der Wickeleinrichtung 105 wird der zweite Wickelkern 106 in einer dafür vorgesehenen Aufnahme positioniert und so ausgerichtet, dass die Matte 102 positionsgenau auf den zweiten Wickelkern 106 aufgewickelt werden kann.

Nach dem Positionieren des zweiten Wickelkerns 106 wird die Matte 102 teilweise vom ersten Wickelkern 104 abgewickelt und zur Spanneinrichtung 112 geführt. Am Anfangsbereich 303 der Matte 102 werden in dieser Ausführungsform zwei bandförmige Führungselemente 304 mit jeweils einem Ende befestigt. Aufgabe der beiden Führungselemente 304 ist es, das positionsgenaue Aufwickeln der Matte 102 auf den zweiten Wickelkern 106 zu unterstützen, indem der Anfangsbereich 303 der Matte 102 und damit auch die gesamte nachfolgende Matte 102 zielgerichtet vom ersten Wickelkern 104 zum zweiten Wickelkern 106 geführt wird. Bei diesen Führungselementen 304 handelt es sich beispielsweise um selbstklebende Folienstreifen. Es können aber auch andere Materialien verwendet werden. Wichtig für die Auswahl des Materials ist auch hier, dass die Matte 102 durch das Ablageelement 109 nicht verunreinigt oder kontaminiert wird. Sowohl die Anzahl als auch die Anordnung der Führungselemente 304 kann dabei variabel gestaltet werden.

Nach dem Befestigen der beiden Führungselemente 304 wird der Anfangsbereich 303 der Matte 102 auf das Ablageelement 109 aufgelegt. Das jeweils andere Ende der beiden Führungselemente 304 wird in Richtung des zweiten Wickelkerns 106 geführt und dort befestigt. Beide Führungselemente 304 werden anschließend teilweise auf den zweiten Wickelkern 106 aufgewickelt, indem der zweite Wickelkern 106 von der ersten Antriebseinheit 124 so lange gedreht wird, bis die Führungselemente 304 gespannt sind. In Verbindung mit der Spanneinrichtung 112 wird so erreicht, dass die Matte 102 gespannt auf dem Ablageelement 109 aufliegt und nicht mehr verrutschen kann.

Zum Ausbilden eines gewickelten Hohlfaserbündels 107 wird nun die Aufnahme mit dem zweiten Wickelkern 106 von der ersten Antriebseinheit 124 so gedreht, dass die Matte 102 vom ersten Wickelkern 104 abgewickelt und mit einer Geschwindigkeit vₘ zum zweiten Wickelkern 106 bewegt und dort aufgewickelt wird. Gleichzeitig mit der Matte 102 wird auch das Ablageelement 109 bewegt, indem der vierte Wickelkern 111 von der zweiten Antriebseinheit 126 so gedreht wird, dass das Ablageelement 109 vom dritten Wickelkern 110 abgewickelt und mit einer Fördergeschwindigkeit v_{f} zum vierten Wickelkern 111 bewegt und dort aufgewickelt wird.

Damit sichergestellt ist, dass die Matte 102 während des Transports auf dem Ablageelement 109 immer gespannt ist und nicht auf dem Ablageelement 109 verrutscht, werden die von der ersten Antriebseinheit 124 erzeugte Drehzahl nₘ und die von der zweiten Antriebseinheit 126 erzeugte Drehzahl n_{f} so von der Regelungseinrichtung 125 aufeinander abgestimmt, dass die Geschwindigkeit vₘ mit der Fördergeschwindigkeit v_{f} synchronisiert ist. Dabei wird bei der Bestimmung der Drehzahlen nₘ und n_{f} unter anderem berücksichtigt, dass die Matte 102 dicker ist als das Ablageelement 109, sodass die Drehbewegungen des zweiten Wickelkerns 106 und des vierten Wickelkerns 111 entsprechend an die unterschiedlich wachsenden Durchmesser angepasst werden müssen.

Während des Transports der Matte 102 auf dem Ablageelement 109 vom ersten Wickelkern 104 zum zweiten Wickelkern 106 wird die Düse 115 der Dosiereinrichtung 113 zum Dosieren einer Vergussmasse entsprechend den Vorgaben der Steuerungseinrichtung 114 so über die Matte 102 geführt, dass auf der Matte 102 mit Vergussmasse benetzte Bereiche bzw. Benetzungskonturen 305, 306 ausgebildet werden, die beispielhaft in Fig. 3b dargestellt sind. Sowohl Form als auch Anzahl der benetzten Bereiche 305, 306 sind dabei variabel und frei wählbar. Mit der Dosiereinrichtung 113 können sowohl gleichförmig benetzte Bereiche, beispielsweise in Form eines Bandes, als auch ungleichförmig benetzte Bereiche beliebiger Form ausgebildet werden. Auch eine Kombination gleichförmig und ungleichförmig benetzter Bereiche ist möglich.

Grundlage für die Festlegung der Form der zu benetzenden Bereiche 305, 306 sind Berechnungen von Strömungsverläufen für den Blutstrom durch das Hohlfasermembranmodul, auf deren Basis Kontur und Anzahl für die vergossenen Bereiche des Hohlfasermembranmoduls festgelegt werden. Ziel dieser Berechnungen ist es, die Blutbestandteile während des Durchströmens des Hohlfasermembranmoduls so wenig wie möglich zu schädigen und den Stoffaustausch zu optimieren. Die Ergebnisse der Strömungsberechnungen bilden dann die Grundlage für die Festlegung der Konturen und Formen der zu benetzenden Bereiche 305, 306, auf denen wiederum die Ansteuerung der Düse 115 der Dosiereinrichtung 113 zur Dosierung der Vergussmasse auf der Matte 102 basiert.

Das ebenfalls in die Dosiereinrichtung 113 integrierte Rakelelement 116 ist nachgeschaltet zur Düse 115 angeordnet, sodass es erst über die Matte 102 geführt wird, wenn diese bereits bereichsweise mit Vergussmasse benetzt ist. Dabei wird das Rakelelement 116 so über die benetzte Matte 102 geführt, dass die Vergussmasse auch in die Zwischenräume eingebracht wird, die zwischen den einzelnen, nebeneinander angeordneten Hohlfasern 301 der Matte 102 ausgebildet werden. Gleichzeitig wird mit dem Rakelelement 116 überschüssige Vergussmasse von der benetzten Matte 102 abgenommen und entfernt. Dadurch erhalten die benetzten Bereiche 305, 306 eine genau definierte, eindeutige Kontur.

Mit dem Aufwickeln der benetzten Matte 102 auf den zweiten Wickelkern 106 in der Wickeleinrichtung 105 lagern sich die benetzten Bereiche 305, 306 lagenweise so übereinander, dass im gewickelten Hohlfaserbündel 107 vergossene Bereiche ausgebildet werden, deren Form und Kontur von der Form der benetzten Bereiche 305, 306 bestimmt wird. Beispielsweise werden aus den benetzten Bereichen 305 vergossene Randbereiche ausgebildet, da sich diese benetzten Bereiche 305 jeweils am Rand der Matte 102 befinden. Aus dem benetzten Bereich 306 hingegen wird durch das Aufwickeln eine Trennschicht im Inneren des gewickelten Hohlfaserbündels 107 ausgebildet. Die vergossenen Randbereiche und Trennschichten sind dabei so ausgebildet, dass Blut und andere Flüssigkeiten schonend durch das Hohlfaserbündel 107 geführt werden können und dass dabei Randgängigkeiten vermieden werden.

Während des Aufwickelns der mit Vergussmasse benetzten Matte 102 auf den zweiten Wickelkern 106 wird durch die Überwachungseinrichtung 127 kontinuierlich der Durchmesser des sich auf dem zweiten Wickelkern ausbildenden Hohlfaserbündels 107 überwacht. Dafür misst beispielsweise ein Sensor den wachsenden Durchmesser des Hohlfaserbündels 107 mit dem zweiten Wickelkern 106. Der Sensor kann aber auch beispielsweise nur die wachsende Wandstärke des Hohlfaserbündels 107 auf dem zweiten Wickelkern 106 messen. Sobald ein für das gewickelte Hohlfaserbündel 107 definierter Durchmesser erreicht ist, leitet die Überwachungseinrichtung 127 ein Signal an die Regelungseinrichtung 125, um die beiden Antriebseinheiten 124, 126 zu stoppen. Dadurch werden sowohl das Abwickeln der Matte 102 und des Ablageelementes 109 als auch der Transport der Matte 102 vom ersten Wickelkern 104 zum zweiten Wickelkern 106 und die Förderung des Ablageelementes 109 vom dritten Wickelkern 110 zum vierten Wickelkern 111 beendet.

Ab- und Aufwickeln von Matte 102 und Ablageelement 109 werden auch dann automatisch beendet, wenn das Ende der Matte 102 oder des Ablageelementes 109 erreicht sind und der jeweils zugehörige Wickelkern leer ist. Eine integrierte Signaleinrichtung erzeugt dann ein entsprechendes Signal, beispielsweise ein akustisches oder visuelles Signal, über das erkennbar ist, dass das Hohlfaserbündel 107 noch nicht fertig gewickelt ist.

Nach dem Stopp des Aufwickelns der benetzten Matte 102 erhält die Trenneinrichtung 117 ein Signal, dass die Matte 102 vom Hohlfaserbündel 107 getrennt werden kann. Damit die Trenneinrichtung 117 die Matte 102 beispielsweise durch Schneiden vom Hohlfaserbündel 107 trennen kann, wird sie aus einer Ausgangsposition in Richtung der Matte 102 verfahren. Anschließend wird die Matte 102 mit der Trenneinrichtung 117 vom Holfaserbündel 107 getrennt und nach dem Trennen wird die Trenneinrichtung 117 wieder in die Ausgangsposition zurück verfahren. Damit sind das Aufwickeln des Hohlfaserbündels 107 und das damit verbundene Ausbilden von vergossenen Bereichen im Hohlfaserbündel 107 abgeschlossen.

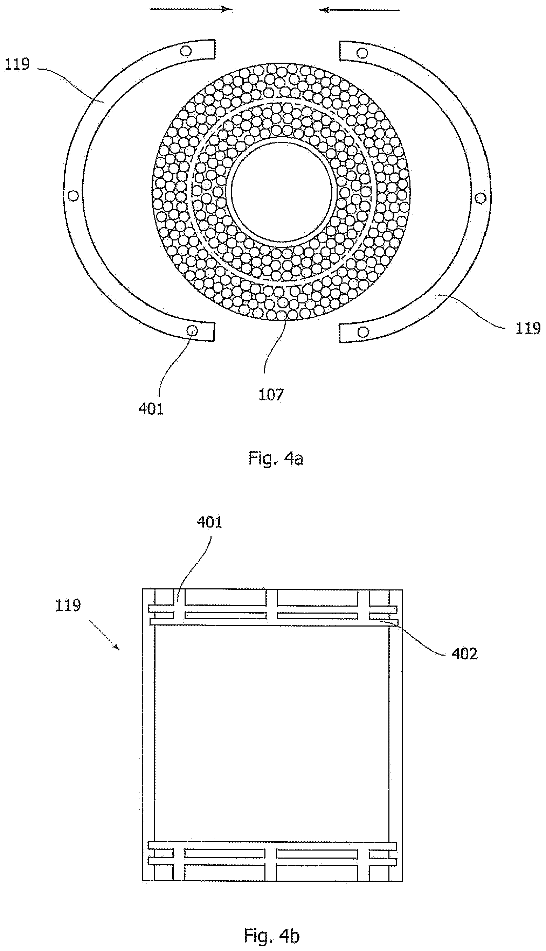

Zur Fertigstellung des Hohlfasermembranmoduls ist jetzt noch ein Gehäuse zu montieren. In Fig. 4a ist schematisch das Hohlfaserbündel 107 mit einem Gehäuse dargestellt, das aus zwei halbschalenförmig ausgebildeten Gehäuseteile 119 zusammengesetzt wird. Die Montage dieser beiden Gehäuseteile 119 erfolgt mit der Montageeinrichtung 118, mit der die beiden Gehäuseteile 119 dem gewickelten Hohlfaserbündel 107 zeitgleich oder auch nacheinander zugeführt werden. Während der Zuführung der beiden Gehäuseteile 119 befindet sich der zweite Wickelkern 106 des Hohlfaserbündels 107 immer noch in der Aufnahme der Wickeleinrichtung 105. Zur Montage werden die beiden Gehäuseteile 119 positionsgenau so um das Hohlfaserbündel 107 herum gelegt, dass ein geschlossenes Gehäuse ausgebildet wird. Die beiden Gehäuseteile 119 werden nach der Zuführung so lange aufeinander zu bewegt, bis ihre Seitenwände Kontakt zueinander haben. Die Bewegung der beiden Gehäuseteile 119 aufeinander zu ist durch zwei Pfeile gekennzeichnet.

Eine feste Verbindung zwischen den beiden Gehäuseteilen 119 wird beispielsweise durch eine Presspassung hergestellt. Weiterhin möglich ist es, eine Klebeverbindung, beispielsweise mittels eines UV-Klebers, oder eine Schraub- oder Nietverbindung zur Verbindung der beiden Gehäuseteile 119 zu verwenden. Alternativ kann das Gehäuse auch als Klappgehäuse mit zwei Gehäuseteilen ausgestaltet sein, das eine Längsteilung aufweist, an der das Gehäuse geöffnet werden kann. Gegenüber dieser Längsteilung ist eine scharnierförmige Verbindung angeordnet, die das Öffnen des Gehäuses realisiert. Möglich ist weiterhin eine Ausgestaltung des Gehäuses mit mehr als zwei Gehäuseteilen, die entweder zeitgleich oder auch nacheinander um das Hohlfaserbündel 107 herum gelegt werden.

Wenn die beiden Gehäuseteile 119 miteinander Kontakt haben, kann das Hohlfaserbündel 107 mit dem montierten Gehäuse entnommen werden. Bei der Entnahme ist darauf zu achten, dass die beiden Gehäuseteile 119 nicht auf dem Hohlfaserbündel 107 verschoben werden. Aus diesem Grund weist die Entnahmeeinrichtung 120 entsprechende Fixierelemente auf, mit denen das Gehäuse während der Entnahme fixiert wird.

Zum Verbinden der beiden Gehäuseteile 119 miteinander ist ein Vergießen mit einer Vergussmasse vorgesehen. Wie in Fig. 4b beispielhaft gezeigt, weisen die beiden Gehäuseteile 119 dafür jeweils im oberen und unteren Bereich jeweils zwei oder mehr Bohrungen 401 auf, in die Vergussmasse eingebracht werden kann und die über jeweils eine oder mehrere Nuten 402 im Innenbereich der Gehäuseteile 119 miteinander verbunden sind. Als vorteilhaft haben sich in beiden Bereichen jeweils 5 Bohrungen und 5 Nuten gezeigt. Es kann aber auch eine andere Anzahl von Bohrungen 401 und Nuten 402 gewählt werden. Das Vergießen der beiden Gehäuseteile 119 erfolgt dann in einer Vergusseinrichtung 121, der das Hohlfaserbündel 107 mit dem montierten Gehäuse mittels der Entnahmeeinrichtung 120 zugeführt wird.

Bevor das Hohlfaserbündel 107 mit dem vergossenen Gehäuse aus der Entnahmeeinrichtung 120 entnommen werden kann, muss die Vergussmasse für das Gehäuse noch aushärten, um ein nachträgliches Verschieben der Gehäuseteile 119 auf dem Hohlfaserbündel 107 zu verhindern. Je nach verwendeter Vergussmasse wird das Hohlfaserbündel 107 mit dem vergossenen Gehäuse dafür entweder Wärme oder auch UV-Licht der Aushärteeinrichtung 122 ausgesetzt.

Nach dem Aushärten der Vergussmasse ist als letzter Schritt noch das Begradigen der vergossenen Randbereiche des Hohlfaserbündels 107 mit der hierfür vorgesehenen Schneideinrichtung 123 erforderlich. Zum Begradigen wird das Messer der Schneideinrichtung 123 aus einer Ausgangsposition an den zu begradigenden, vergossenen Randbereich des Hohlfaserbündels 107 heran geführt und dann an diesem in Richtung des zweiten Wickelkerns 106 entlang geführt. Während dieser Bewegung werden die Hohlfasern 301 aufgeschnitten und einzelne Hohlfasern 301, die aus dem vergossenen Randbereich des Hohlfaserbündels 107 herausragen, werden entsprechend dabei abgeschnitten. Wenn die Schneideinrichtung 123 den zweiten Wickelkern 106 erreicht hat, wird sie wieder in ihre Ausgangsposition zurück verfahren.

Damit ist die Herstellung eines Hohlfasermembranmoduls abgeschlossen und das fertige Hohlfasermembranmodul kann aus der Entnahmeeinrichtung 120 entnommen werden.

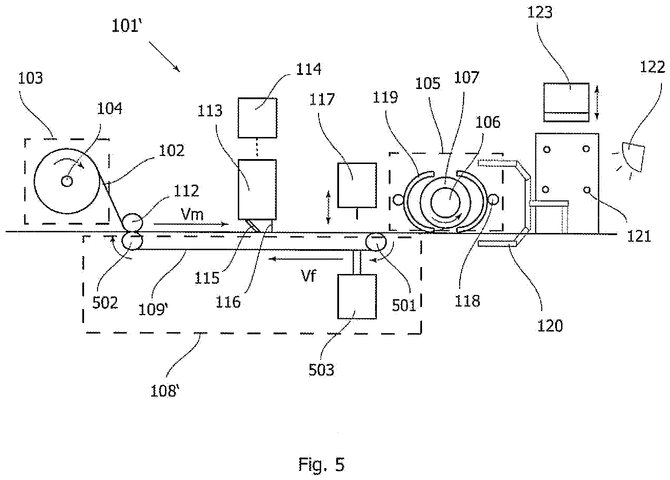

Die Seitenansicht einer anderen Vorrichtung 101' zur Herstellung eines Hohlfasermembranmoduls aus einer bandförmigen Matte 102 ist in Fig. 5 schematisch als Beispiel dargestellt. Der Unterschied zur Vorrichtung 101 besteht insbesondere in der Fördereinrichtung 108'. In dieser Fördereinrichtung 108' ist das Ablageelement 109' als Endlosband ausgebildet, das von einer Antriebswelle 501 angetrieben werden kann und um eine weitere Welle 502 geführt wird. Angetrieben wird die Antriebswelle 501 dabei von der zweiten Antriebseinheit 126.

Da das Ablageelement 109' in dieser Ausführungsform als Endlosband ausgebildet ist, wurde zur Vermeidung von Verunreinigungen der nicht benetzten Matte 102 eine Reinigungseinrichtung 503 zur Reinigung des Ablageelementes 109' in die Vorrichtung 101' integriert. Diese Reinigungsvorrichtung 503 reinigt das Ablageelement 109' nach der Abnahme der benetzten Matte 102 und beseitigt die auf dem Ablageelement 109' anhaftenden Reste der Vergussmasse, die während des Benetzens durch die Matte 102 auf das Ablageelement 109' gelangt sind. Damit steht das Ablageelement 109' nach der Reinigung für die nicht benetzte Matte 102 wieder in gereinigtem Zustand zur Verfügung.

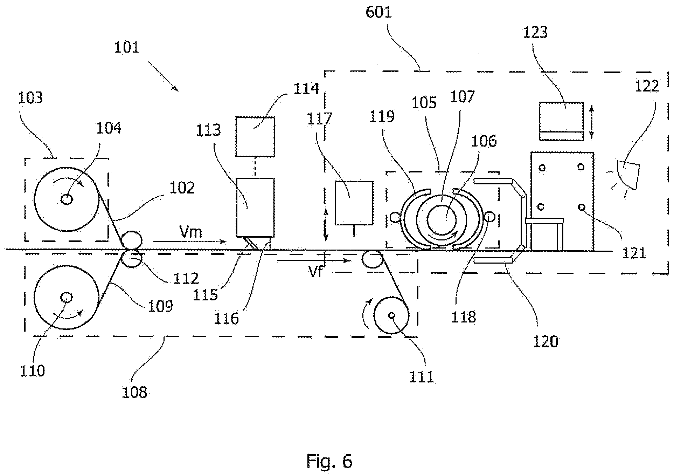

Erweitert werden können sowohl die Vorrichtung 101 als auch die Vorrichtung 101' mit einer Kammer 601, die beispielhaft in Fig. 6 dargestellt ist und in die ein nicht dargestellter Niederdruck-Plasmagenerator integriert ist. Mit dem Plasmagenerator wird in der Kammer 601 eine Atmosphäre erzeugt, in der das Hohlfaserbündel 107 schon während des Aufwickelns sterilisiert wird. Werden in die Kammer 601 auch alle nachfolgenden Einrichtungen zur Fertigstellung des Hohlfasermembranmoduls, wie beispielsweise die Montageeinrichtung 118 oder die Vergusseinrichtung 121 sowie eine nicht dargestellte Verpackungseinrichtung, integriert werden und läuft das gesamte Verfahren vollautomatisch ab, kann das fertiggestellte und verpackte Hohlfasermembranmodul sofort verwendet werden, da es bereits sterilisiert ist. Eine Ausgasungszeit wie bei der Verwendung von EO als Sterilisationsgas ist hierbei nicht erforderlich.

### Bezugszeichenliste:

- 101, 101': Vorrichtung
- 102: Matte aus Hohlfasern
- 103: Zuführeinrichtung
- 104: erster Wickelkern
- 105: Wickeleinrichtung
- 106: zweiter Wickelkern
- 107: Hohlfaserbündel
- 108, 108': Fördereinrichtung
- 109, 109': Ablageelement
- 110: dritter Wickelkern
- 111: vierter Wickelkern
- 112: Spanneinrichtung
- 113: Dosiereinrichtung
- 114: Steuerungseinrichtung
- 115: Düse
- 116: Rakelelement
- 117: Trenneinrichtung
- 118: Montageeinrichtung
- 119: Gehäuseteil
- 120: Entnahmeeinrichtung
- 121: Vergusseinrichtung
- 122: Aushärteeinrichtung
- 123: Schneideinrichtung
- 124: erste Antriebseinheit
- 125: Regelungseinrichtung
- 126: zweite Antriebseinheit
- 127: Überwachungseinrichtung
- 201: erste Aufnahme
- 202: Magnet
- 203: zweite Aufnahme
- 204: Zentrierelement
- 205: erstes Kernteil
- 206: zweites Kernteil
- 207: Einlasskontur
- 208: drittes Kernteil, Rotor
- 209: Montagestift
- 210: Montagehilfe

- 301: Hohlfaser
- 302: Verbindungsfaden
- 303: Anfangsbereich der Matte
- 304: Führungselement
- 305: Benetzungskontur, benetzter Bereich
- 306: Benetzungskontur, benetzter Bereich

- 401: Bohrung
- 402: Nut

- 501: Antriebswelle
- 502: Welle
- 503: Reinigungseinrichtung

- 601: Kammer

## Patentansprüche

1. Verfahren zur Herstellung eines Hohlfasermembranmoduls aus mindestens einer bandförmigen Matte (102) mit einzelnen, nebeneinander angeordneten Hohlfasern (301), und auf einen zweiten, zylinderförmigen Wickelkern (106) so aufgewickelt wird, dass ein gewickeltes Hohlfaserbündel (107) ausgebildet wird, wobei der zweite Wickelkern (106) aus mindestens einem Teil gebildet wird, umfassend folgende Schritte:
• Transport der Matte (102) von dem ersten Wickelkern (104) in Richtung des zweiten Wickelkerns (106) mit einer variierbaren Geschwindigkeit vₘ, wobei die Matte (102) auf einem bandförmigen Ablageelement (109, 109') aufliegt, das mit einer variierbaren Fördergeschwindigkeit vₘ bewegt wird, und wobei die Geschwindigkeit vₘ mit der Fördergeschwindigkeit v_{f} synchronisiert wird; sowie
• Bereichsweises Benetzen der Matte (102) mit einer Vergussmasse während des Transports der Matte (102) auf dem Ablageelement (109, 09'), wobei auf der benetzten Matte (102) mindestens eine gleichförmige und/oder ungleichförmige Benetzungskontur (305, 306) ausgebildet wird,
wobei das Verfahren folgende kennzeichnende weitere Schritte umfasst:
• Abwickeln der Matte (102) von einem ersten, zylinderförmigen Wickelkern (104),
• Montieren eines ersten Kernteiles (205) des zweiten Wickelkerns (106) auf eine erste Aufnahme (201), wobei die erste Aufnahme (201) einen Magneten (202) aufweist;
• Montieren eines zweiten Kernteiles (206) des zweiten Wickelkerns (106) auf eine zweite Aufnahme (203), wobei die zweite Aufnahme (203) ein Zentrierelement (204) aufweist;
• wobei das erste Kernteil (205) und das zweite Kernteil (206) als Röhre ausgebildet sind;
• wobei das Zentrierelement (204) eine Zentrierspitze aufweist, um das zweite Kernteil (206) über das Zentrierelement (204) auf der zweiten Aufnahme (203) zu zentrieren,
• das zweite Kernteil im Inneren eine Einlasskontur (207) mit einer Bohrung aufweist, die zur Zentrierung des zweiten Kernteils (206) auf dem Zentrierelement (204) verwendet wird,
• das erste Kernteil (205) an einem Ende verschlossen ist und auf der ersten Aufnahme (201) das erste Kernteil (205) so montiert ist, dass das geschlossene Ende über dem Magneten (202) angeordnet ist und das dritte Kernteil (208) positionsgenau zwischen de ersten Kernteil (205) und dem zweiten Kernteil (206) angeordnet ist,
• Positionieren eines dritten Kernteiles (208) des zweiten Wickelkerns (106) auf der ersten Aufnahme (201), wobei der dritte Kernteil (208) magnetisch ist und auf dem Magneten (202) der ersten Aufnahme (201) haftet;
• Positionieren der zweiten Aufnahme (203) gegenüber der ersten Aufnahme (201), wobei beide Aufnahmen axial ausgerichtet werden können,
• wobei, nachdem die einzelnen Kernteile des zweiten Wickelkerns (106) auf der Aufnahme positioniert ausgerichtet sind, die zweite Aufnahme (203) in Richtung der ersten Aufnahme (201) verfahren wird, bis die Zentrierspitze das dritte Kernteil (208) berührt, und dann axial ausgerichtet wird.

2. Verfahren nach Anspruch 1, umfassend folgende weitere Schritte:
• Verdichten der Vergussmasse auf der Matte (102) und gleichzeitiges Entfernen überschüssiger Vergussmasse von der Matte (102);
• Abnehmen der benetzten Matte (102) von dem Ablageelement (109, 109');
• Aufwickeln der benetzten Matte (102) auf den zweiten Wickelkern (106), wobei die erste Aufnahme (201) von einer ersten Antriebseinheit (124) angetrieben wird und wobei die zweite Aufnahme (203) mit einer Drehzahl gedreht wird, die mit der von der ersten Antriebseinheit (124) erzeugten Drehzahl der ersten Aufnahme (201) synchronisiert ist;
• Überwachen bestimmter Maße des Hohlfaserbündels (107) während des Aufwickelns der benetzten Matte (102);
• Stoppen des Aufwickelns, wenn das gewickelte Hohlfaserbündel (107) definierte Abmessungen aufweist; sowie
• Trennen des gewickelten Hohlfaserbündels (107) von der Matte (102).

3. Verfahren nach einem der vorherigen Ansprüche, umfassend folgende weitere Schritte:
• Montieren eines hohlzylinderförmigen Gehäuses um das gewickelte Hohlfaserbündel (107), wobei das Gehäuse mindestens zwei zumindest bereichsweise voneinander getrennte Gehäuseteile (119) umfasst, die das Hohlfaserbündel (107) nach dem Montieren zumindest bereichsweise umschließen und wobei das Gehäuse an beiden Enden offen ist;
• Entnahme des Hohlfaserbündels (107) mit dem Gehäuse, wobei die beiden Gehäuseteile (119) fixiert werden;
• Vergießen der beiden Gehäuseteile (119) mit Vergussmasse und Aushärten der Vergussmasse;
• Begradigen beider Randbereiche des gewickelten Hohlfaserbündels (107), sodass alle Hohlfasern (301) jeweils offene Enden aufweisen.

4. Verfahren nach einem der vorherigen Ansprüche, umfassend folgende weitere Schritte vor dem Transport der Matte (102):
• Befestigen eines Endes von mindestens einem bandförmigen Führungselement (304) an dem zweiten Wickelkern (106);
• Befestigen des anderen Endes des mindestens einen Führungselementes (304) an einem Anfangsbereich (303) der Matte (102);
• Auflegen der Matte (102) auf das Ablageelement (109, 109');
• Vorspannen der Matte (102) und zumindest teilweises Aufwickeln des Führungselementes (304) auf den zweiten Wickelkern (106); sowie
• Abwickeln der Matte (102) von dem ersten Wickelkern (104).

5. Verfahren nach einem der vorherigen Ansprüche, bei dem das Aufwickeln der benetzten Matte (102) in einer Kammer (601) unter einer von einem Niederdruck-Plasmagenerator erzeugten Atmosphäre erfolgt, sodass das Hohlfaserbündel (107) während des Aufwickelns gleichzeitig sterilisiert wird.

6. Verfahren nach Anspruch 5, bei dem das gewickelte und sterilisierte Hohlfaserbündel (107) automatisch in der Kammer (601) in das Gehäuse montiert und steril verpackt wird.

7. Verfahren nach einem der vorherigen Ansprüche, bei dem das Auflageelement (109) von einem dritten, zylinderförmigen Wickeikern (110) abgewickelt und auf einen vierten, zylinderförmigen Wickelkern (111) aufgewickelt oder über eine Andruckrolle weitergefördert wird, wobei der vierte Wickelkern (111) oder die Andruckrolle von einer zweiten Antriebseinheit (126) angetrieben wird.

8. Verfahren nach einem der Ansprüche 1 bis 6, bei dem das Auflageelement (109') als Endlosband von mindestens einer Antriebswelle (501) bewegt und um mindestens eine weitere Welle (502) geführt wird, wobei das Auflageelement (109') nach der Abnahme der benetzten Matte (102) gereinigt wird.

9. Vorrichtung (101, 101') zur Herstellung eines Hohlfasermembranmoduls aus mindestens einer bandförmigen Matte (102) mit einzelnen, nebeneinander angeordneten Hohlfasern (301), umfassend:
• eine Fördereinrichtung (108, 108'), die so ausgebildet ist, dass sie ein bandförmiges Ablageelement (109, 109') mit einer variierbaren Fördergeschwindigkeit v_{f} fördern kann, wobei die Matte (102) auf dem Ablageelement (109, 109') aufliegen kann;
• eine Dosiereinrichtung (113) für Vergussmasse, die so ausgebildet ist, dass sie die Matte (102) während eines Transports so bereichsweise benetzen kann, dass auf der Matte (102) mindestens eine gleichförmige und/oder ungleichförmige Benetzungskontur (305, 306) ausbildbar ist;
• eine Wickeleinrichtung (105), die so ausgebildet ist, dass sie die benetzte Matte (102) mit einer variierbaren Geschwindigkeit vₘ von einem ersten Wickelkern (104) auf einen zweiten Wickelkern (106) aufwickeln kann, sodass ein gewickeltes Hohlfaserbündel (107) ausgebildet wird, wobei der zweite Wickelkern (106) aus mindestens einem Teil gebildet wird; sowie
• eine Regelungseinrichtung (125), die so ausgebildet ist, dass sie die Geschwindigkeit vₘ mit der Fördergeschwindigkeit v_{f} synchronisieren kann, wobei die Vorrichtung (101, 101'), weiterhin umfasst:
• eine erste Aufnahme (201), die so ausgebildet ist, dass sie ein erstes Kernteil (205) des zweiten Wickelkerns (106) aufnehmen kann, wobei die erste Aufnahme (201) einen Magneten (202) aufweist, der so ausgebildet ist, dass er ein drittes Kernteil (208) des zweiten Wickelkerns (106) aufnehmen kann; und
• eine zweite Aufnahme (203), die so ausgebildet ist, dass sie ein zweites Kernteil (206) des zweiten Wickelkerns (106) aufnehmen kann, wobei die zweite Aufnahme (203) ein Zentrierelement (204) aufweist und wobei die zweite Aufnahme (203) über ein Getriebe mit der ersten Aufnahme (201) so verbunden ist, dass die erste und zweite Aufnahme synchronisiert drehbar sind;
• wobei das Zentrierelement (204) eine Zentrierspitze aufweist, um das zweite rohrförmige Kernteil (206) über das Zentrierelement (204) auf der zweiten Aufnahme (203) zu zentrieren, wobei das zweite Kernteil im Inneren eine Einlasskontur (207) mit einer Bohrung aufweist,
• der Magnet so ausgebildet ist, dass das erste rohrförmige Kernteil (205), das an einem Ende verschlossen ist, so auf der ersten Aufnahme (201) des ersten Kernteils (205) montiert werden kann, dass das geschlossene Ende des ersten Kernteils über dem Magneten angeordnet ist, und
• dass die zweite Aufnahme (203) gegenüber der ersten Aufnahme (201) positioniert werden kann, wobei die zweite Aufnahme (203) in Richtung der ersten Aufnahme (201) verfahren werden kann bis die Zentrierspitze das dritte Kernteil (208) berührt und dann axial ausgerichtet wird, sodass das dritte Kernteil (208) positionsgenau zwischen dem ersten Kernteil (205) und dem zweiten Kernteil (206) angeordnet werden kann..

10. Vorrichtung (101, 101') nach Anspruch 9, weiterhin umfassend:
• eine Überwachungseinrichtung (127), die so ausgebildet ist, dass sie bestimmte Maße des Hohlfaserbündels (107) während des Aufwickelns der benetzten Matte (102) überwachen kann und dass sie das Aufwickeln stoppen kann, wenn das gewickelte Hohlfaserbündel (107) definierte Abmessungen aufweist; sowie
• eine Trenneinrichtung (117), die so ausgebildet ist, dass sie das gewickelte Hohlfaserbündel (107) von der Matte (102) trennen kann.

11. Vorrichtung (101, 101') nach einem der Ansprüche 9 oder 10, weiterhin umfassend;
• eine Montageeinrichtung (118), die so ausgebildet ist, dass sie ein hohlzylinderförmiges Gehäuse um das gewickelte Hohlfaserbündel (107) montieren kann, wobei das Gehäuse mindestens zwei zumindest bereichsweise voneinander getrennte Gehäuseteile (119) umfasst, die das Hohlfaserbündel (107) nach der Montage zumindest bereichsweise umschließen und wobei das Gehäuse an beiden Enden offen ist;
• eine Entnahmeeinrichtung (120), die so ausgebildet ist, dass sie das Hohlfaserbündel (107) mit dem Gehäuse entnehmen kann;
• eine Vergusseinrichtung (121) für Vergussmasse, die so ausgebildet ist, dass sie die beiden Gehäuseteile (119) des Gehäuses vergießen kann;
• eine Aushärteeinrichtung (122), die so ausgebildet ist, dass sie die Vergussmasse aushärten kann; sowie
• eine Schneideinrichtung (123), die so ausgebildet ist, dass sie beide Randbereiche des gewickelten Hohlfaserbündels (107) so begradigen kann, dass alle Hohlfasern (301) jeweils offene Enden aufweisen.

12. Vorrichtung (101, 101') nach einem der Ansprüche 9 bis 11, weiterhin umfassend:
• eine Zufuhreinrichtung (103), die so ausgebildet ist, dass sie die Matte von einem ersten Wickelkern (104) abwickeln kann; sowie
• eine Spanneinrichtung (112), die so ausgebildet ist, dass sie die Matte (102) vorspannen kann.

13. Vorrichtung (101, 101') nach einem der Ansprüche 9 bis 12, bei der die Dosiereinrichtung (113) ein Rakelelement (116) aufweist, das so ausgebildet ist, dass es die Vergussmasse auf der Matte (102) verdichten und gleichzeitig überschüssige Vergussmasse von der Matte (102) entfernen kann.

14. Vorrichtung (101, 101') nach einem der Ansprüche 9 bis 13, bei der die Überwachungseinrichtung (127) einen Sensor zur Messung eines Durchmessers des gewickelten Hohlfaserbündels (107) aufweist.

15. Vorrichtung (101, 101') nach einem der Ansprüche 9 bis 14, bei der die Entnahmeeinrichtung (120) zangenförmig ausgebildet ist und mindestens ein Fixierelement zum Fixieren der beiden Gehäuseteile (119) während der Entnahme aufweist.

16. Vorrichtung (101, 101') nach einem der Ansprüche 9 bis 15, bei der die Wickeleinrichtung (105) in einer Kammer (601) angeordnet ist, wobei die Kammer (601) so ausgebildet ist, dass in der Kammer mittels eines Niederdruck-Plasmagenerators eine Atmosphäre zum Sterilisieren des Hohlfaserbündels (107) während des Aufwickelns erzeugbar ist.

17. Vorrichtung (101, 101') nach Anspruch 16, bei der in der Kammer (601) eine automatische Montageeinrichtung (118) und eine automatische Verpackungseinrichtung für das gewickelte und sterilisierte Hohlfaserbündel (107) angeordnet ist.

18. Hohlfasermembranmodul, das nach einem Verfahren gemäß den Ansprüchen 1 bis 8 hergestellt wurde.

## Claims

1. Method for the production of a hollow fibre membrane module from at least one band-form mat (102) with individual hollow fibres (301) arranged next to one another, and which is wound onto a second, cylindrical winding core (106) such that a wound hollow fibre bundle (107) is formed, wherein the second winding core (106) is formed from at least one part, comprising the following steps:
• Transport of the mat (102) from the first winding core (104) in the direction of the second winding core (106) at a variable speed vₘ, wherein the mat (102) rests on a band-form deposition element (109, 109'), which is moved at a variable conveying speed vₘ, and wherein the speed vₘ is synchronised with the conveying speed v_{f}; and
• Region-wise wetting of the mat (102) with a casting compound during the transport of the mat (102) on the deposition element (109, 09'), wherein at least one uniform and/or non-uniform wetting contour (305, 306) is formed on the wetted mat (102),
wherein the method comprises the following further characterising steps:
• Unwinding of the mat (102) from a first cylindrical winding core (104),
• Mounting of a first core part (205) of the second winding core (106) onto a first receptacle (201), wherein the first receptacle (201) has a magnet (202);
• Mounting of a second core part (206) of the second winding core (106) onto a second receptacle (203), wherein the second receptacle (203) has a centring element (204); wherein
• The first core part (205) and the second core part (206) are formed as tubes; wherein
• The centring element (204) has a centring tip for purposes of centring the second core part (206) via the centring element (204) on the second receptacle (203),
• The interior of the second core part has an inlet contour (207) with a bore, which is used to centre the second core part (206) on the centring element (204),
• The first core part (205) is closed at one end, and the first core part (205) is mounted on the first receptacle (201) such that the closed end is arranged above the magnet (202), and the third core part (208) is accurately arranged in a position between the first core part (205) and the second core part (206),
• Positioning of a third core part (208) of the second winding core (106) on the first receptacle (201), wherein the third core part (208) is magnetic and adheres to the magnet (202) of the first receptacle (201);
• Positioning of the second receptacle (203) opposite the first receptacle (201), wherein both receptacles can be axially aligned, wherein,
• After the individual core parts of the second winding core (106) are aligned, positioned on the receptacle, the second receptacle (203) is driven in the direction of the first receptacle (201) until the centring tip contacts the third core part (208), and is then axially aligned.

2. Method according to Claim 1, comprising the following further steps:
• Compression of the casting compound on the mat (102) and the simultaneously removal of excess casting compound from the mat (102);
• Removal of the wetted mat (102) from the deposition element (109, 109');
• Winding of the wetted mat (102) onto the second winding core (106), wherein the first receptacle (201) is driven by a first drive unit (124), and wherein the second receptacle (203) is rotated at a speed that is synchronised with the speed of the first receptacle (201) generated by the first drive unit (124);
• Monitoring of certain dimensions of the hollow fibre bundle (107) during the winding of the wetted mat (102)
• Halting of the winding when the wound hollow fibre bundle (107) has defined dimensions; and
• Separation of the wound hollow fibre bundle (107) from the mat (102).

3. Method according to one of the preceding claims, comprising the following further steps:
• Mounting of a hollow cylindrical housing around the wound hollow fibre bundle (107), wherein the housing comprises at least two housing parts (119) which are separated from one another at least in some regions, and which enclose the hollow fibre bundle (107) at least in some regions after the mounting, and wherein the housing is open at both ends;
• Removal of the hollow fibre bundle (107) with the housing, wherein the two housing parts (119) are fixed;
• Casting of the two housing parts (119) with casting compound, and curing of the casting compound;
• Straightening of both edge regions of the wound hollow fibre bundle (107), such that all hollow fibres (301) have respective open ends.

4. Method according to one of the preceding claims, comprising the following further steps before the transport of the mat (102):
• Attachment of one end of at least one band-form guide element (304) to the second winding core (106);
• Attachment of the other end of the at least one guide element (304) to an initial region (303) of the mat (102);
• Placement of the mat (102) onto the deposition element (109, 109');
• Prestressing of the mat (102) and at least partial winding of the guide element (304) onto the second winding core (106); together with
• Unwinding of the mat (102) from the first winding core (104).

5. Method according to one of the preceding claims, in which the winding of the wetted mat (102) takes place in a chamber (601) under an atmosphere generated by a low-pressure plasma generator, such that the hollow fibre bundle (107) is simultaneously sterilised during the winding.

6. Method according to Claim 5, in which the wound and sterilised hollow fibre bundle (107) is automatically mounted in the chamber (601) in the housing, and is sterile packed.

7. Method according to one of the preceding claims, in which the deposition element (109) is unwound from a third, cylindrical winding core (110) and wound onto a fourth, cylindrical winding core (111), or conveyed onward by way of a pressure roller, wherein the fourth winding core (111), or the pressure roller, is driven by a second drive unit (126).

8. Method according to one of the Claims 1 to 6, in which the deposition element (109) is moved as an endless belt by at least one drive shaft (501), and is guided around at least one further shaft (502), wherein the deposition element (109') is cleaned after the removal of the wetted mat (102).

9. Device (101, 101') for the production of a hollow fibre membrane module from at least one band-form mat (102) with individual hollow fibres (301) arranged side by side, comprising:
• Conveying device (108, 108'), which is designed such that it can convey a band-form deposition element (109, 109') at a variable conveying speed v_{f}, wherein the mat (102) can lie on the deposition element (109, 109');
• A metering device (113) for the casting compound, which is designed such that it can wet the mat (102) in some regions during transport, such that at least one uniform and/or non-uniform wetting contour (305, 306) can be formed on the mat (102);
• A winding device (105), which is designed so as to wind the wetted mat (102) at a variable speed vₘ from a first winding core (104) onto a second winding core (106), so as to form a wound hollow fibre bundle (107), wherein the second winding core (106) is formed from at least one part; together with
• A control device (125), which is designed so as to synchronise the speed vₘ with the conveying speed v_{f}, wherein the device (101, 101'), further comprises:
• A first receptacle (201), which is designed such that it can receive a first core part (205) of the second winding core (106), wherein the first receptacle (201) has a magnet (202), which is designed such that it can receive a third core part (208) of the second winding core (106); and
• A second receptacle (203), which is designed such that it can receive a second core part (206) of the second winding core (106), wherein the second receptacle (203) has a centring element (204), and wherein the second receptacle (203) is connected to the first receptacle (201) by way of a transmission, such that the first and second receptacles can be rotated synchronously; wherein
• The centring element (204) has a centring tip, so as to centre the second tubular core part (206) over the centring element (204) on the second receptacle (203), wherein the interior of the second core part has an inlet contour (207) with a bore,
• The magnet is designed such that the first tubular core part (205), which is closed at one end, can be mounted on the first receptacle (201) of the first core part (205) such that the closed end of the first core part is arranged above the magnet, and
• That the second receptacle (203) can be positioned opposite the first receptacle (201), wherein the second receptacle (203) can be driven in the direction of the first receptacle (201) until the centring tip contacts the third core part (208) and is then axially aligned, such that the third core part (208) can be accurately arranged in a position between the first core part (205) and the second core part (206).

10. Device (101, 101') according to Claim 9, further comprising:
• A monitoring device (127), which is designed such that it can monitor certain dimensions of the hollow fibre bundle (107) during the winding of the wetted mat (102), and can halt the winding when the wound hollow fibre bundle (107) has defined dimensions; together with
• A separating device (117), which is designed such that it can separate the wound hollow fibre bundle (107) from the mat (102).

11. Device (101, 101') according to one of the Claims 9 or 10, further comprising:
• An assembly device (118), which is designed such that it can assemble a hollow cylindrical housing around the wound hollow fibre bundle (107), wherein the housing comprises at least two housing parts (119), which are separated from one another at least in some regions, and which enclose the hollow fibre bundle (107) at least in some regions after assembly, and wherein the housing is open at both ends;
• A removal device (120), which is designed such that it can remove the hollow fibre bundle (107) with the housing;
• A casting device (121) for casting compound, which is designed such that it can cast the two housing parts (119) of the housing;
• A curing device (122), which is designed such that it can cure the casting compound; together with
• A cutting device (123), which is designed such that it can straighten both edge parts of the wound hollow fibre bundle (107), such that all the hollow fibres (301) have respectively open ends.

12. Device (101, 101') according to one of the Claims 9 to 11, further comprising:
• A feeding device (103), which is designed such that it can unwind the mat from a first winding core (104); together with
• tensioning device (112), which is designed such that it can pretension the mat (102).

13. Device (101, 101') according to one of the Claims 9 to 12, in which the metering device (113) has a squeegee element (116), which is designed such that it can compact the potting compound on the mat (102), while simultaneously removing excess casting compound from the mat (102)

14. Device (101, 101') according to one of the Claims 9 to 13, in which the monitoring device (127) has a sensor for purposes of measuring a diameter of the wound hollow fibre bundle (107).

15. Device (101, 101') according to one of the Claims 9 to 14, in which the removal device (120) is designed in the form of tongs, and has at least one fixing element for purposes of fixing the two housing parts (119) during the removal.

16. Device (101, 101') according to one of the Claims 9 to 15, in which the winding device (105) is arranged in a chamber (601), wherein the chamber (601) is designed such that an atmosphere for sterilising the hollow fibre bundle (107) during winding can be generated in the chamber by means of a low-pressure plasma generator.

17. Device (101, 101') according to Claim 16, in which an automatic assembly device (118) and an automatic packaging device for the wound and sterilised hollow fibre bundle (107) are arranged in the chamber (601).

18. Hollow fibre membrane module, which has been produced by a method according to the Claims 1 to 8.

## Revendications

1. Procédé de production d'un module membranaire à fibres creuses à partir d'au moins un tapis en forme de bande (102) comportant des fibres creuses individuelles juxtaposées (301), lequel module membranaire est enroulé sur un deuxième noyau d'enroulement de forme cylindrique (106) de manière à constituer un faisceau enroulé de fibres creuses (107), le deuxième noyau d'enroulement (106) étant composé d'au moins une partie, comprenant les étapes suivantes :
• transport du tapis (102) du premier noyau d'enroulement (104) en direction du deuxième noyau d'enroulement (106) à une vitesse variable vm, le tapis (102) reposant sur un élément de dépôt en forme de bande (109, 109') qui est déplacé à une vitesse de convoyage variable vm, la vitesse vm étant synchronisée avec la vitesse de convoyage vf ; et
• par endroits, imbibage du tapis (102) avec une masse coulée pendant le transport du tapis (102) sur l'élément de dépôt (109, 109'), au moins un contour d'imbibage uniforme et/ou non uniforme (100, 306) étant constitué sur le tapis imbibé (102),
le procédé comprenant les autres étapes de caractérisation suivantes :
• déroulement du tapis (102) hors d'un premier noyau d'enroulement de forme cylindrique (104),
• montage d'une première partie de noyau (205) du deuxième noyau d'enroulement (106) sur un premier support (201), le premier support (201) présentant un aimant (202) ;
• montage d'une deuxième partie de noyau (206) du deuxième noyau d'enroulement (106) sur un second support (203), le second support (203) présentant un élément de centrage (204) ;
• la première partie de noyau (205) et la deuxième partie de noyau (206) étant réalisées sous forme de tubes ;
• l'élément de centrage (204) présentant une pointe de centrage pour centrer la deuxième partie de noyau (206) au-dessus de l'élément de centrage (204) sur le second support (203) ;
• la deuxième partie de noyau présentant à l'intérieur un contour d'introduction (207) doté d'un alésage qui est utilisé pour centrer la deuxième partie de noyau (206) sur l'élément de centrage (204),
• la première partie de noyau (205) étant fermée à une extrémité et la première partie de noyau (205) étant montée sur le premier support (201) de manière à ce que l'extrémité fermée soit disposée au-dessus de l'aimant (202) et que la troisième partie de noyau (208) soit disposée à une position précise entre la première partie de noyau (205) et la deuxième partie de noyau (206),
• positionnement d'une troisième partie de noyau (208) du deuxième noyau d'enroulement (106) sur le premier support (201), la troisième partie de noyau (208) étant magnétique et adhérant sur l'aimant (202) du premier support (201) ;
• positionnement du second support (203) en face du premier support (201), les deux supports pouvant être orientés axialement,
• dans lequel procédé, une fois que les parties individuelles de noyau du deuxième noyau d'enroulement (106) sont orientées positionnées sur le support, le second support (203) est déplacé en direction du premier support (201) jusqu'à ce que la pointe de centrage touche la troisième partie de noyau (208) et soit ensuite orientée axialement.

2. Procédé selon la revendication 1, comprenant les autres étapes suivantes :
• épaississement de la masse coulée sur le tapis (102) et élimination simultanée de la masse coulée excédentaire du tapis (102) ;
• retrait du tapis imbibé (102) de l'élément de dépôt (109, 109') ;
• enroulement du tapis imbibé (102) sur le deuxième noyau d'enroulement (106), le premier support (201) étant entraîné par une première unité motrice (124) et le second support (203) tournant à une vitesse de rotation qui est synchronisée avec la vitesse de rotation générée par la première unité motrice (124) du premier support (201) ;
• surveillance de certaines dimensions du faisceau de fibres creuses (107) pendant l'enroulement du tapis imbibé (102) ;
• arrêt de l'enroulement une fois que le faisceau de fibres creuses enroulé (107) présente des dimensions définies ; et
• séparation du faisceau de fibres creuses enroulé (107) et du tapis (102).

3. Procédé selon une des revendications précédentes, comprenant les autres étapes opératoires suivantes :
• montage d'un boîtier en forme de cylindre creux autour du faisceau de fibres creuses enroulé (107), le boîtier comprenant au moins deux parties de boîtier (119) séparées l'une de l'autre du moins par endroits et qui entourent du moins par endroits le faisceau de fibres creuses (107) après le montage, et le boîtier étant ouvert aux deux extrémités ;
• enlèvement du faisceau de fibres creuses (107) avec le boîtier, les deux parties du boîtier (119) étant fixées ;
• coulage des deux parties du boîtier (119) avec une masse coulée et durcissement de la masse coulée ;
• arrangement des deux zones périphériques du faisceau de fibres creuses enroulé (107) de manière à ce que toutes les fibres creuses (301) présentent respectivement des extrémités ouvertes.

4. Procédé selon une des revendications précédentes, comprenant les autres étapes opératoires suivantes avant le transport du tapis (102) :
• fixation d'une extrémité d'au moins un élément de guidage en forme de bande (304) sur le deuxième noyau d'enroulement (106) ;
• fixation de l'autre extrémité de l'au moins un élément de guidage (304) à une partie initiale (303) du tapis (102) ;
• pose du tapis (102) sur l'élément de dépôt (109, 109') ;
• précontrainte du tapis (102) et enroulement au moins partiel de l'élément de guidage (304) sur le deuxième noyau d'enroulement (106) ; et
• déroulement du tapis (102) hors du premier noyau d'enroulement (104).

5. Procédé selon une des revendications précédentes, dans lequel l'enroulement du tapis imbibé (102) a lieu dans une chambre (601) sous une atmosphère générée par un générateur de plasma à basse pression, de sorte que le faisceau de fibres creuses (107) est en même temps stérilisé pendant l'enroulement.

6. Procédé selon la revendication 5, dans lequel le faisceau de fibres creuses enroulé et stérilisé (107) est monté automatiquement dans le boîtier dans la chambre (601) et placé sous emballage stérile.

7. Procédé selon une des revendications précédentes, dans lequel l'élément support (109) est déroulée hors d'un troisième noyau d'enroulement de forme cylindrique (110) et enroulé sur un quatrième noyau d'enroulement de forme cylindrique (111) ou est transporté plus loin par un rouleau à compression, le quatrième noyau d'enroulement (111) ou le rouleau à compression étant entraîné par une seconde unité motrice (126).

8. Procédé selon une des revendications 1 à 6, dans lequel l'élément support (109') est déplacé sous forme d'une bande continue par au moins un arbre moteur (501) et guidé autour d'au moins un autre arbre (502), l'élément support (109') étant nettoyé après l'enlèvement du tapis imbibé (102).

9. Dispositif (101, 101') de production d'un module membranaire à fibres creuses à partir d'au moins un tapis en forme de bande (102) comportant des fibres creuses individuelles juxtaposées (301), comprenant :
• un dispositif de transport (108, 108') qui est réalisé de manière à pouvoir transporter un élément de dépôt en forme de bande (109, 109') à une vitesse de convoyage variable vf, le tapis (102) pouvant reposer sur l'élément de dépôt (109, 109') ;
• un dispositif de dosage (113) pour la masse coulée, qui est réalisé de manière à pouvoir imbiber le tapis (102) par endroits pendant un transport de manière à ce qu'au moins un contour d'imbibage uniforme et/ou non uniforme (305, 306) puisse être réalisé sur le tapis (102) ;
• un dispositif d'enroulement (105) qui est réalisé de manière à pouvoir enrouler le tapis imbibé (102) à une vitesse variable vm depuis un premier noyau d'enroulement (104) sur un deuxième noyau d'enroulement (106) de manière à constituer un faisceau de fibres creuses enroulé (107), le deuxième noyau d'enroulement (106) étant constitué d'au moins une partie ; et
• un dispositif de réglage (125) qui est réalisé de manière à pouvoir synchroniser la vitesse vm avec la vitesse de convoyage vf, le dispositif (101, 101') comprenant en outre :
• un premier support (201) qui est réalisé de manière à pouvoir recevoir une première partie de noyau (205) du deuxième noyau d'enroulement (106), le premier support (201) présentant un aimant (202) qui est réalisé de manière à pouvoir recevoir une troisième partie de noyau (208) du deuxième noyau d'enroulement (106) ; et
• un second support (203) qui est réalisé de manière à pouvoir recevoir une deuxième partie de noyau (206) du deuxième noyau d'enroulement (106), le second support (203) présentant un élément de centrage (204) et le second support (203) étant connecté par une transmission au premier support (201) de manière à ce que les premier et second supports puissent tourner de manière synchronisée ;
• l'élément de centrage (204) présentant une pointe de centrage pour centrer la deuxième partie de noyau de forme tubulaire (206) au-dessus de l'élément de centrage (204) sur le second support (203), la deuxième partie de noyau présentant à l'intérieur un contour d'introduction (207) doté d'un alésage,
• l'aimant étant réalisé de manière à ce que la première partie de noyau de forme tubulaire (205) qui est fermée à une extrémité puisse être montée sur le premier support (201) de la première partie de noyau (205) de manière à ce que l'extrémité fermée de la première partie de noyau soit disposée au-dessus de l'aimant, et
• le second support (203) pouvant être positionné en face du premier support (201), le second support (203) pouvant être déplacé en direction du premier support (201) jusqu'à ce que la pointe de centrage touche la troisième partie de noyau (208) et soit ensuite orientée axialement de manière à ce que la troisième partie de noyau (208) puisse être disposée à une position précise entre la première partie de noyau (205) et la deuxième partie de noyau (206).

10. Dispositif (101, 101') selon la revendication 9, comprenant en outre :
• un dispositif de surveillance (127) qui est conçu pour pouvoir surveiller certaines dimensions du faisceau de fibres creuses (107) pendant l'enroulement du tapis imbibé (102) et pour pouvoir arrêter l'enroulement lorsque le faisceau de fibres creuses enroulé (107) présente des dimensions définies ; et
• un dispositif de séparation (117) qui est conçu pour pouvoir séparer le faisceau de fibres creuses enroulé (107) du tapis (102).

11. Dispositif (101, 101') selon une des revendications 9 ou 10, comprenant en outre :
• un dispositif de montage (118) qui est conçu pour pouvoir monter un boîtier en forme de cylindre creux autour du faisceau de fibres creuses enroulé (107), le boîtier présentant au moins deux parties de boîtier séparées l'une de l'autre du moins par endroits (119) et qui entourent le faisceau de fibres creuses (107) au moins par endroits après le montage, le boîtier étant ouvert aux deux extrémités ;
• un dispositif d'enlèvement (120) qui est conçu pour pouvoir enlever le faisceau de fibres creuses (107) avec le boîtier ;
• un dispositif de coulage (121) pour de la masse coulée, qui est réalisé de manière à pouvoir couler les deux parties de boîtier (119) du boîtier ;
• un dispositif de durcissement (122) qui est conçu pour pouvoir durcir la masse coulée ; et
• un dispositif de coupe (123) qui est conçu pour arranger les deux zones périphériques du faisceau de fibres creuses enroulé (107) de manière à ce que toutes les fibres creuses (301) présentent respectivement des extrémités ouvertes.

12. Dispositif (101, 101') selon une des revendications 9 à 11, comprenant en outre :
• un dispositif d'acheminement (103) qui est conçu pour pouvoir dérouler le tapis hors d'un premier noyau d'enroulement (104); et
• un dispositif tendeur (112) qui est conçu pour pouvoir précontraindre le tapis (102).

13. Dispositif (101, 101') selon une des revendications 9 à 12, dans lequel le dispositif de dosage (113) présente un élément de raclage (116) qui est conçu pour pouvoir épaissir la masse coulée sur le tapis (102) et en même temps éliminer la masse coulée excédentaire du tapis (102).

14. Dispositif (101, 101') selon une des revendications 9 à 13, dans lequel le dispositif de surveillance (127) présente un capteur pour mesurer un diamètre du faisceau de fibres creuses enroulé (107).

15. Dispositif (101, 101') selon une des revendications 9 à 14, dans lequel le dispositif d'enlèvement (120) est réalisé en forme de pince et présente au moins un élément de fixation pour la fixation des deux parties de boîtier (119) pendant l'enlèvement.

16. Dispositif (101, 101') selon une des revendications 9 à 15, dans lequel le dispositif d'enroulement (105) est disposé dans une chambre (601), la chambre (601) étant réalisée de manière à ce que, dans la chambre, au moyen d'un générateur de plasma à basse pression, une atmosphère de stérilisation du faisceau de fibres creuses (107) puisse être générée pendant l'enroulement.

17. Dispositif (101, 101') selon la revendication 16, dans lequel un dispositif de montage automatique (118) et un dispositif d'emballage automatique pour le faisceau de fibres creuses enroulé et stérilisé (107) est disposé dans la chambre (601).

18. Module membranaire à fibres creuses qui a été produit par un procédé selon les revendications 1 à 8.
